(19) Europäisches Patentamt / European Patent Office / Office européen des brevets

(11) EP 2 400 033 B1

(12) **EUROPEAN PATENT SPECIFICATION**

(45) Date of publication and mention of the grant of the patent:
**20.02.2013 Bulletin 2013/08**

(51) Int Cl.:
***C12Q 1/48*** *(2006.01)* ***G01N 33/574*** *(2006.01)*

(21) Application number: **11170125.6**

(22) Date of filing: **16.06.2011**

(54) **Method for determining sensitivity of tumor cells to tyrosine kinase inhibitor and computer program product**

Verfahren zur Bestimmung der Empfindlichkeit von Tumorzellen auf einen Tyrosinkinaseinhibitor und Computerprogrammprodukt

Procédé pour déterminer la sensibilité de cellules tumorales à un inhibiteur de la tyrosine kinase et produit de programme informatique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2010 JP 2010142831**
**28.01.2011 JP 2011016759**

(43) Date of publication of application:
**28.12.2011 Bulletin 2011/52**

(73) Proprietor: **SYSMEX CORPORATION**
**Kobe-shi,**
**Hyogo 651-0073 (JP)**

(72) Inventors:
- **Kawai, Masaaki**
  **Hyogo 651-0073 (JP)**
- **Torikoshi, Yasuhiro**
  **Hyogo 651-0073 (JP)**
- **Gohda, Keigo**
  **Hyogo 651-0073 (JP)**

(74) Representative: **HOFFMANN EITLE**
**Patent- und Rechtsanwälte**
**Arabellastrasse 4**
**81925 München (DE)**

(56) References cited:
**WO-A2-2007/106432** **WO-A2-2008/089135**
**WO-A2-2008/127718** **WO-A2-2010/009171**

- **SONG L ET AL: "Dasatinib (BMS-354825) selectively induces apoptosis in lung cancer cells dependent on epidermal growth factor receptor signaling for survival", CANCER RESEARCH, AMERICAN ASSOCIATION FOR CANCER RESEARCH, US, vol. 66, no. 11, 1 June 2006 (2006-06-01), pages 5542-5548, XP002558116, ISSN: 0008-5472, DOI: 10.1158/0008-5472.CAN-05-4620**
- **NAUTIYAL JYOTI ET AL: "Src inhibitor dasatinib inhibits growth of breast cancer cells by modulating EGFR signaling", CANCER LETTERS, NEW YORK, NY, US, vol. 283, no. 2, 8 October 2009 (2009-10-08), pages 143-151, XP002597452, ISSN: 0304-3835, DOI: 10.1016/J.CANLET.2009.03.035 [retrieved on 2009-04-26]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to a method for determining sensitivity of tumor cells to tyrosine kinase inhibitor and a computer program product. More particularly, the present invention relates to a method for determining sensitivity of the tumor cells to an EGFR and/or HER2 inhibitor which is tyrosine kinase different from Src by evaluating inhibitory capacity of a tyrosine kinase Src inhibitor in a sample for measuring tyrosine kinase activity which is prepared from a biological sample containing tumor cells and a computer program product.

BACKGROUND

Tyrosine kinase is a molecule which is involved in signaling for regulating cell differentiation and growth. Abnormalities of the tyrosine kinase are known to be causes of diseases such as cancer. The expression level of tyrosine kinase and its activity are excessively enhanced, particularly in tumor cells of various types of cancer, such as breast cancer and colon cancer and it is known that growth potential of tumor cells is activated by the excessive enhancement. Thus, a molecular target drug for targeting tyrosine kinase and inhibiting its activity has been studied and developed.

Examples of the target molecule for such a drug include Epidermal Growth Factor Receptor (EGFR; also referred to as "HER1") and Human Epidermal growth factor Receptor 2 (HER2). EGFR is a receptor-type tyrosine kinase. If its ligand, i.e., an epidermal growth factor (EGF) is bound to EGFR, an EGF signal which controls cell growth and differentiation is transmitted. HER2 is a molecule cloned as a receptor-type tyrosine kinase similar to human EGFR and regulates cell growth and differentiation similarly to EGFR.

Examples of inhibitors using EGFR and HER2 as target molecules include lapatinib, i.e., an inhibitor antagonistic to adenosine triphosphate (ATP) which is a phosphate group donor. Since such an inhibitor is generally a low molecular weight compound, it passes through a tumor cell membrane and binds directly and reversibly to an ATP binding site of EGFR and HER2 in the cell, thereby inhibiting auto-phosphorylation of EGFR and HER2. Accordingly, the signaling of tyrosine kinases of EGFR and HER2 is inhibited. As a result, the growth of tumor cells is suppressed, and further apoptosis of the cells is induced.

Currently, the inhibitor of EGFR and HER2 including lapatinib is confirmed to be effective for cancer with excessive expression of EGFR and HER2.

However, an ATP competitive inhibitor for EGFR and HER2 is not always effective for all cancer. For example, the effectiveness of lapatinib is specifically observed in HER2 positive breast cancer. Therefore, in order to decide to treat a certain cancer patient with the inhibitor of EGFR and HER2, it is necessary to predict whether the inhibitor seems to be effective for the cancer patient by a test.

At present, examples of the test method include an immunohistochemical staining method (IHC method) which includes examining expression levels of EGFR and HER2 proteins in tumor cells obtained from patients and a fluorescent in situ hybridization method (FISH method) which includes examining amplification of EGFR and HER2 genes. As the results of these tests, when it is determined that there are high expression levels of EGFR and HER2 proteins and/or there is gene amplification, it is assumed that the inhibitor of EGFR and HER2 is effective for the patient. However, in these tests, variations in results are easily caused depending on characteristics of antibodies and probes to be used and the tester's technique, and thus it is difficult to perform accurate tests.

As another procedure for assuming the effects of the inhibitor of EGFR and HER2, there is a method described in the reference of Gottfried E. Konecny et al. (Gottfried E. Konecny et al., Cancer Res, vol.66, p.1630-1639(2006)). In the reference, effects of lapatinib are evaluated by culturing cells derived from breast cancer in the presence of lapatinib and examining growth inhibition effects on the cells. However, it takes a long time to obtain results because the method is accompanied by a step of culturing cells. In order to take out tumor cells from biological samples such tissues taken from patients and culture them equally, a skillful technique is required. Since variations in results being obtained are easily caused depending on measurement environment, it is difficult to perform accurate tests.

Experimental results of tyrosine kinase activity inhibitory effects on 13 types of cell lines caused by the Src inhibitor are shown in US 2011027809 which discloses a method for evaluating malignancy in tumor cells. Here, Examples of US 2011027809 suggest that, in the case of two cell lines (BT-474 and SK-Br-3) which are lapatinib sensitive lines are disclosed in the reference of Gottfried E. Konecny et al. , tyrosine kinase activity inhibitory effects caused by the Src inhibitor is high, meanwhile, in the case of three cell lines (MDA-MB231, MCF7, and T47D) which are lapatinib insensitive lines are disclosed in the reference of Konecny et al., tyrosine kinase activity inhibitory effects caused by the Src inhibitor is low.

WO 2008/127718 provides methods for predicting the sensitivity of tumor cell growth to inhibition by an EGFR kinase inhibitor, comprising assessing whether the tumor cell has undergone an epithelial to mesenchymal transition, by determining the expression level of epithelial and/or mesenchymal biomarkers like Src.

WO 2007/106432 relates to a method for predicting the response of a subject having a disease or condition mediated by EGFR to an EGFR kinase inhibitor. The method comprises measuring the amount of phosphorylation at residues Y1068 and T1148 in EGFR in a sample from the subject, wherein a significantly elevated level of phosphorylation at the two residues compared to a baseline value indicates that the subject is likely to be responsive to an agent that inhibits the kinase activity of EGFR.

WO 2008/089135 discloses a method of predicting response to treatment with inhibitors of EGFR and Src by screening for status of key biomarkers such as EGFR.

Song et al. evaluated the effectiveness of the Src inhibitor desatinib in lung cancer cell lines with defined EGFR status and found that in cell with EGFR mutation that are dependent on EGFR for survival, desatinib reduces cell viability through the induction of apoptosis while having minimal apoptotic effect on cell lines with wild-type EGFR (Lanxi Song et al., Cancer Res, vol.66, p.5542-5548(2006)).

SUMMARY OF THE INVENTION

The scope of the present invention is defined solely by the appended claims, and is not affected to any degree by the statements within this summary.

In view of the above circumstances, an object of the present invention is to provide a method for determining sensitivity of tumor cells to an inhibitor of EGFR and HER2 which does not need to have a cell culture process and does not need to examine the expression level of protein of tyrosine kinase and/or gene amplification and a computer program product.

Surprisingly, the present inventors have found a negative correlation between the sensitivity of tumor cells to the inhibitor of EGFR and HER2 and the inhibitory capacity of the inhibitor of tyrosine kinase Src in a sample for measuring tyrosine kinase activity prepared from the tumor cells. That is, they have found out that the inhibitory capacity of Src inhibitor was low in a measurement sample prepared from tumor cells which had been previously confirmed to be sensitive to the inhibitor of EGFR and HER2, meanwhile, the inhibitory capacity of Src inhibitor was high in a measurement sample prepared from tumor cells which had been previously confirmed to be insensitive to the inhibitor of EGFR and HER2 and completed the present invention.

Src is a type of non-receptor tyrosine kinase which is unevenly distributed in the body, i.e., tyrosine kinase distinct from EGFR and HER2.

A first aspect of the present invention is a method for determining sensitivity of tumor cells to an ATP competitive inhibitor for EGFR and/or HER2 comprising steps of: evaluating inhibitory capacity of an ATP competitive inhibitor for tyrosine kinase Src in a measurement sample obtained by preparing a cellular fraction containing tyrosine kinase from a biological sample containing tumor cells; and determining that the tumor cells have sensitivity to an ATP competitive inhibitor for tyrosine kinase EGF and/or HER2 when the inhibitory capacity of the ATP competitive inhibitor for Src is evaluated to be low and/or determining that the tumor cells do not have sensitivity to the ATP competitive inhibitor to EGFR and/or HER2 when the inhibitory capacity of the ATP competitive inhibitor for Src is evaluated to be high.

A second aspect of the present invention is a computer program product comprising: a computer readable medium, and software instructions, on the computer readable medium, for enabling a computer to perform operations comprising: acquiring an activity value of tyrosine kinase in a measurement sample obtained from a biological sample containing tumor cells in the presence of an ATP competitive inhibitor for tyrosine kinase Src; calculating a value of inhibitory capacity of the ATP competitive inhibitor for Src based on the activity value; comparing the value of inhibitory capacity of the ATP competitive inhibitor for Src with a threshold value; determining that the tumor cells have sensitivity to the ATP competitive inhibitor for tyrosine kinase EGFR and/or HER2 when the value of inhibitory capacity of the ATP competitive inhibitor for Src is lower than the threshold value based on the compared result and/or determining that the tumor cells do not have sensitivity to the ATP competitive inhibitor for EGFR and/or HER2 when the value of inhibitory capacity of the ATP competitive inhibitor for Src is more than the threshold value; and outputting determination results.

A third aspect of the present invention is a method for determining effectiveness of an ATP competitive inhibitor on EGFR and/or HER2 in a cancer patient comprising steps of:

> evaluating inhibitory capacity of an ATP competitive inhibitor for tyrosine kinase Src in a measurement sample obtained by preparing a cellular fraction containing tyrosine kinase from a biological sample containing tumor cells collected from the cancer patient; and determining that the ATP competitive inhibitor for tyrosine kinase EGFR and/or HER2 is effective for the cancer patient when the inhibitory capacity of the ATP competitive inhibitor to Src is evaluated to be low and/or determining that the ATP competitive inhibitor for tyrosine kinase EGFR and/or HER2 is not effective for the cancer patient when the inhibitory capacity of the ATP competitive inhibitor for Src is evaluated to be high.

The sensitivity of tumor cells to the ATP competitive inhibitor for EGFR/HER2 can be determined highly accurately and simply by measuring tyrosine kinase activity regarding a measurement sample obtained from a biological sample containing tumor cells and evaluating levels of inhibitory capacity of the ATP competitive inhibitor for Src in the sample according to the determination method.

According to the computer program product, it is possible to allow a computer to perform the determination method.

Based on the determination result obtained by the determination method, a doctor in clinical practice can obtain a more accurate indicator to determine whether the ATP competitive inhibitor for EGFR/HER2 such as lapatinib should be administered to the cancer patient in whom the biological sample is collected.

BRIEF DESCRIPTION OF THE DRAWINGS

Fig. 1 is a block diagram showing a hardware configuration of a system for determining sensitivity of an ATP competitive inhibitor to EGFR/HER2;
Fig. 2 is a flow chart showing a process of determining sensitivity of tumor cells to the ATP competitive inhibitor for EGFR/HER2 by a CPU 110a;
Fig. 3 shows graphs showing inhibition rates of various types of kinase inhibitors in a lapatinib-sensitive cell line group and a lapatinib-insensitive cell line group; and
Fig. 4 is a photograph showing expression of the HER2 protein in various types of breast cancer cell lines obtained by Western blotting.

DETALLED DESCRIPTION OF THE PREFERRED EMBODIMENTS

In the present specification, the term "sensitivity of tumor cells to the ATP competitive inhibitor for EGFR/HER2" means characteristics of tumor cells in which the survival and/or growth of tumor cells is suppressed or inhibited by effects of the ATP competitive inhibitor on EGFR/HER2.

That is, the survival and/or growth of tumor cells with sensitivity to the ATP competitive inhibitor for EGFR/HER2 is substantially suppressed or inhibited by the inhibitor. On the other hand, the survival and/or growth of tumor cells without such sensitivity is not substantially suppressed or inhibited by the inhibitor. In this regard, mechanisms in which the survival and/or growth of tumor cells is suppressed or inhibited by the effects of the ATP competitive inhibitor on EGPR/HER2 include induction of cell death, such as apoptosis and necrosis.

The ATP competitive inhibitor for EGFR/HER2 (hereinafter also referred to as an "EGFR/HER2 inhibitor") is not particularly limited as long as it is a substance which inhibits an enzyme activity of EGFR, and/or HER2 by binding to an adenosine triphosphate (ATP) binding site of EGFR and/or HER2. Examples of the EGFR/HER2 inhibitor include the following compounds: - N-(3-chloro-4-[[(3-fluorophenyl)methyl]oxy]phenyl)-6-[5-([[2-(methylsulfonyl)ethyl]amino]methyl)-2-furanyl]-4-quina zolineamine bis(4-methylbenzenesulfonate)monohydrate (hereinafter referred to as "lapatinib ditosylate hydrate"); - N-[3-chloro-4-[(3-fluorophenyl) methoxy]phenyl]-6-[5-[(2-methylsulfonylethylamino)methyl]-2

- furyl]quinazolin-4-amine (hereinafter referred to as "lapatinib" or "GW572016");
- N-[4-[(3chloro-4-fluorophenyl)amino]-7-[[(3S)-tetrahydro-3-furanyl]oxy]-6-quinazolinyl]-4-(dimethylamino)
- 2-butenamide (hereinafter referred to as "BIBW 2992");
- 6-[4-[(4-ethyl-1-piperazinyl)methyl]phenyl]-N-[(1S)-1-phenylethyl]-7H-pyrrolo[2,3-d]pyrimidin-4-amine (hereinafter referred to as "AEE788");
- (2E)-N-[4-[[3-chloro-4-(2-pyridinylmethoxy)phenyl]amino]-3-Cyano-7-ethoxy-6-quinolinyl]-4-(dimethylamino)-2-butenami de (hereinafter referred to as "neratinib" or "HKI-272");
- [4-[[1-(3-fluorophenyl)methyl]-1H-indazol-5-ylamino]-5-methylpyrrolo[2,1-f][1,2,4]triazine-6-yl]carbamic acid, (3S)-3-morpholinylmethylester (hereinafter referred to as "BMS-599626");
- N4-[3-chloro-4-(thiazol-2-ylmethoxy)phenyl]-N6-[(4R)-4-methyl-4,5-dihydrooxazol-2-yl]quinazoline-4,6-diamine (hereinafter referred to as "varlitinib" or "ARRY-334543");
- 4-(4-benzyloxyanilino)-6,7-dimethoxyquinazoline (hereinafter referred to as "4557W");
- N4-(1-benzyl-1H-indazol-5-yl)-N6,N6-dimethyl-pyrido-[3,4-d]-pyririmidine-4,6-diamine (hereinafter referred to as "GW2974"); and
- N-[3-chloro-4-[(3-fluorophenyl)methoxy]phenyl]-6-[2-[[[2-(methylsulfonyl)ethyl]amino]methyl]-4-thiazolyl]-4-qui nazolineamine dihydrochloride (hereinafter referred to as "GW583340").

The method for determining sensitivity of tumor cells to the ATP competitive inhibitor for EGFR/HER2 in the present embodiment (hereinafter also referred to as the determination method of the present embodiment) is particularly

suitable for determination of sensitivity of tumor cells to lapatinib ditosylate hydrate and lapatinib. All of the EGFR/HER2 inhibitors are known and commonly-available. The CAS number of the EGFR/HER2 inhibitors are shown in Table 1.

[Table 1]

| Name | CAS No. |
|---|---|
| Lapatinib ditosylate hydrate | **388082-78-8** |
| Lapatinib (GW572016) | **231277-92-2** |
| **BIBW 2992** | **439081-18-2** |
| **AEE788** | **1155336-34-7** |
| Neratinib (HKI-272) | **698387-09-6** |
| **BMS-599626** | **714971-09-2** |
| Varlitinib (ARRY-334543) | **845272-21-1** |
| **4557W** | **202272-68-2** |
| **GW2974** | **388082-81-3** |
| **GW583340** | **627518-40-5** |

In the determination method of the present embodiment, a sample for measuring tyrosine kinase activity is obtained by preparing a cellular fraction containing tyrosine kinase from a biological sample containing tumor cells.

The biological sample containing tumor cells may be a sample derived from a biological specimen or a sample derived from a culture obtained by culturing established tumor cells. Examples of the biological specimen include tissues (tumor tissues, organ tissues, lymph node tissues, blood and the like) obtained from biological body (e.g. patients under consideration for cancer treatment with EGFR/HER2 inhibitor) or celomic lavage fluid.

The tumor cells are not particularly limited as long as they are tumor cells which are known to be involved in abnormalities of EGFR and/or HER2, such as breast cancer, lung cancer, gastric cancer, colon cancer, kidney cancer, ovarian cancer, prostatic cancer, oral cancer, liver cancer, skin cancer, and brain cancer cells. The breast cancer cells are preferred.

The cellular fraction containing tyrosine kinase can be prepared from the biological sample containing tumor cells by a known method in itself in the art. Such a method is not particularly limited as long as it is a method for obtaining a cellular fraction which is contained in a state where the enzyme activity of tyrosine kinase in the cells is not impaired. Preferably, the preparation is performed by a method for separating cytoplasm from the biological sample containing tumor cells, namely, a method for separating the cell membrane fraction to which tyrosine kinase is bound from cytoplasm. More preferably, the preparation is performed by a method including homogenizing the biological sample containing tumor cells in an appropriate buffer (hereinafter referred to as a "homogenizing reagent"), obtaining an insoluble fraction from the thus obtained homogenate, mixing the insoluble fraction with a solubilizing solution containing a surfactant, and obtaining a soluble fraction from the obtained mixture.

The homogenate obtained from the homogenization using the homogenizing reagent can be separated into a soluble fraction (e.g. supernatant) and an insoluble fraction (e.g. pellet) by an appropriate method such as centrifugation. The soluble fraction contains cytoplasm-derived proteins and the like and the insoluble fraction contains cell membrane fragments which have various types of tyrosine kinases including EGFR, HER2, and Src.

The mixture can be separated into a soluble fraction (e. g. supernatant) and an insoluble fraction (e.g. pellet) by an appropriate method such as centrifugation. The soluble fraction contains cell membranes having various tyrosine kinases which are solublized with the surfactant (in micelles), and the insoluble fraction contains insoluble proteins, DNA and the like.

The homogenization of cells can be performed by a method for fragmenting a cell membrane. Examples of the method include known methods in the art, such as aspiration and discharge with a pipette, cell homogenization by freezing and thawing, mixing by a vortex mixer, homogenization with a blender, pressurization with a pestle, sonication with a sonicator.

The homogenizing reagent may be used for preventing denaturation of tyrosine kinase during the homogenization of cells. The pH of the homogenizing reagent is not particularly limited so long as it is within a range in which tyrosine kinase can be recovered in a stable state without being denatured or deactivated. The pH of the homogenizing reagent is preferably from pH 4.0 to 9.0, more preferably from pH 4.5 to 8.5, still more preferably from pH 5.0 to 8.0.

Preferably, the homogenizing reagent contains a buffer. Examples of the buffer include a phosphoric acid buffer,

an acetic acid buffer, a citric acid buffer, MOPS (3-morpholinopropanesulfonic acid), HEPES(2-[4-(2-hydroxyethyl)-1-piperazinyl]ethane sulfonic acid), TRIS(tris(hydroxymethyl)aminomethane), and TRICINE(N-[tris(hydroxymethyl)methyl] glycine).

The surfactant contained in the solubilized liquid is not particularly limited as long as it can solubilize the fragmented cell membrane into micelles and does not degrade and denature tyrosine kinase contained in the cell membrane. Since a surfactant with a charge may be bound to tyrosine kinase and change the conformation, it is preferable to use a nonionic surfactant which is not substantially bound to tyrosine kinase. Examples of the nonionic surfactant include surfactants having dodecylether, cetyl ether, stearyl ether, and p-t-octyl phenyl ether as a basic structure. Specific examples thereof include Nonidet P-40 (registered trademark of NP-40, Shell International Petroleum Company Limited), Triton-X (registered trademark of Union Carbide Chemicals and Plastics Inc.), Tween (registered trademark of ICI Americas Inc.), Brij (registered trademark of ICI Americas Inc.) and Emulgon (registered trademark of Kao Corporation.). The concentration of the surfactant in the solubilized liquid is usually from 0.05 to 5%, preferably from 0.1 to 3%, more preferably from 0.1 to 1%.

Preferably, the solubilized liquid contains the same buffer to be used for the homogenizing reagent. Further, the solubilized liquid preferably has a pH equal to that of the homogenizing reagent.

The homogenizing reagent and the solubilized liquid may contain a protease inhibitor, a phosphatase inhibitor, a reagent for preventing the oxidization of a mercapto group (SH group) (hereinafter referred to as an "SH group stabilizer") or the like.

The protease inhibitor can be used in order to prevent the degradation of tyrosine kinases by proteases contained in cells. Examples of the protease inhibitor include metalloprotease inhibitors such as ethylenediaminetetraacetic acid (EDTA) and glycoletherdiaminetetraacetic acid (EGTA); serine protease inhibitors such as phenylmethylsulfonyl fluoride (PMSF), a trypsin inhibitor, and chymotrypsin; and cysteine protease inhibitors such as iodoacetamide and E-64. These protease inhibitors may be used alone or mixed with multiple inhibitors for use. A commercial product such as Protease Inhibitor Cocktail (Sigma) may be used, in which multiple protease inhibitors are premixed.

The phosphatase inhibitor can be used in order to prevent the decrease in tyrosine kinase activities due to phosphatases contained in cells. Examples of the phosphatase inhibitor include sodium orthovanadate (Na3VO4), sodium fluoride (NaF), and okadaic acid. These phosphatase inhibitors may be used alone or mixed with multiple inhibitors for use.

The SH group stabilizer can be used in order to prevent deactivation of tyrosine kinases. SH groups of enzymes are liable to be oxidized to form stable disulfides. The formation of disulfides may result in deactivation of enzymes due to the change in the enzyme conformation. Examples of the SH group stabilizer for preventing such oxidation of SH groups include reagents containing an SH group. Specific examples thereof include dithiothreitol (DTT), 2-mercaptoethanol, glutathione, cysteine, homocysteine, coenzyme A, and dihydrolipoic acid.

The concentration of the SH group stabilizer in the homogenizing reagent and/or solubilizing solution is usually from 0.05 to 2 mM, preferably from 0.07 to 1.7 mM, more preferably from 0.1 to 1.5 mM in case of DTT, for example. It is usually from 0.1 to 15 mM, preferably from 0.3 to 13 mN, more preferably 0.5 to 12 mM in case of 2-mercaptoethanol, for example.

As for the prepared cellular fraction containing tyrosine kinase, the receptor-type tyrosine kinase can form a polymer like homo- and hetero-dimers and can include tyrosine kinase held by the cell membrane in a state where the conformation is maintained at a degree that Src can form a complex with the polymer and/or in a state where Src is anchored to the cell membrane by a lipid chain added to a domain to be lipid-activated which is contained in Src. Preferably, the cellular fraction contains a receptor-type tyrosine kinase with kinase activity and an Src kinase.

In the determination method of the present embodiment, the cellular fraction containing tyrosine kinase prepared in the above manner may be used as a sample for measuring enzyme activities of various types of tyrosine kinases contained in tumor cells.

The determination method of the present embodiment evaluates inhibitory capacity of the ATP competitive inhibitor for tyrosine kinase Src in the measurement sample.

In the present specification, the term "inhibitory capacity of the ATP competitive inhibitor for Src" means a degree that the enzyme activity of tyrosine kinase contained in the measurement sample to be described later is inhibited by the inhibitor.

The ATP competitive inhibitor for Src (hereinafter also referred to as an "Src inhibitor") is not particularly limited as long as it is a substance which inhibits the enzyme activity of Src by binding to the ATP binding site of Src. Examples of the Src inhibitor include the following compounds;

- 4-(4'-phenoxyanilino)-6,7-dimethoxyquinazoline (hereinafter hereinafter referred to as "Src kinase inhibitor 1") ;
- 1-(1,1-dimethylethyl)-1-(4-methylphenyl)-1H-pyrazolo-[3,4-d]pyrimidine-4-amine (hereinafter referred to as "PP1") ;
- 4-amino-1-tert-butyl-3-(1'-naphthyl)pyrazolo[3,4-d] pyrimidine (hereinafter referred to as "PP1 analog");
- 4-amino-5-(4-chlorophenyl)-7-(t-butyl)pyrazolo[3,4-d] pyrimidine (hereinafter referred to as "PP2" or "AG1879") ; and

- 2-oxo-3-(4,5,6,7-tetrahydro-1H-indole- 2-yl methylene)-2,3-dihydro-1H-indole-5-sulfonic acid dimethylamide (hereinafter referred to as "SU6656"); and
- a compound represented by Structural formula (I) below (hereinafter referred to as "Src kinase inhibitor 2").

Structural formula (I)

[Formula 1]

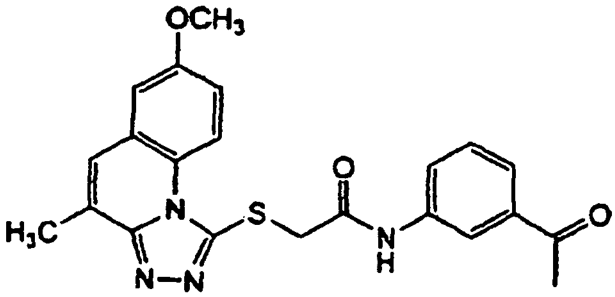

In the determining method of the present embodiment, the Src inhibitors may be used alone or may be mixed with multiple inhibitors for use. Preferably, certain Src inhibitors are used alone. Among the Src inhibitors, the Src kinase inhibitor 1 is preferably used. These Src inhibitors are marketed and commonly-available. The manufacturer and CAS number of the Src inhibitors are shown in Table 2 .

[Table 2]

| **Name** | CAS No. | Manufacturer |
|---|---|---|
| **Src** Kinase inhibitor 1 | **179248-59-0** | Calbiochem Corp. |
| **PP1** | **172889-26-8** | Biomol Corp. |
| **PP1** Analog | **221243-82-9** | Calbiochem Corp. |
| **PP2(AG1879)** | **172889-27-9** | Calbiochem Corp. |
| **SU6656** | **330161-87-0** | Calbiochem Corp. |
| **src** Kinase inhibitor 2 | None | Calbiochem Corp. |

Specifically, the inhibitory capacity of the Src inhibitor in the measurement sample is evaluated based on the activity value which is obtained by measuring the activity value of tyrosine kinase in the measurement sample in the presence of the Src inhibitor. Preferably, the inhibitory capacity of the Src inhibitor is evaluated based on the activity values which are obtained by measuring the activity value of tyrosine kinase in the measurement sample in the absence of the inhibitor, and measuring in the presence or absence of the inhibitor. In this case, the inhibitory capacity of the Src inhibitor is preferably evaluated based on a difference or a ratio of a tyrosine kinase activity value measured in the presence of the Src inhibitor (hereinafter also referred to as a "first activity value") and a tyrosine kinase activity value measured in the absence of the Src inhibitor (hereinafter also referred to as a "second activity value") regarding the measurement sample obtained by the preparation.

Examples of the value of the difference between the first and second activity values include a value obtained by subtracting the second activity value from the first activity value and a value obtained by subtracting the first activity value from the second activity value. Examples of the ratio of the first and second activity values include a value obtained by dividing the first activity value by the second activity value and a value obtained by dividing the second activity value by the first activity value. The ratio of the first and the second activity values may be the inhibition rate calculated by using the value obtained by dividing the first activity value by the second activity value, i.e., a value (%) of an equation: "100-{(the first activity value)/(the second activity value)}x100".

The method for measuring the tyrosine kinase activity value is not particularly limited as long as it is a known method. The method for measuring the activity value of tyrosine kinase includes, for example, a method for detecting the phosphorylation of tyrosine kinases. Specifically, cells are incubated, tyrosine kinase is recovered from cell membranes of the cultured cells, and auto-phosphorylation is detected using a phosphorylated tyrosine recognizing antibody which is labeled with a radioactive substance. Alternatively, the activity value of tyrosine kinase may be measured using a commercially available kit for performing the above method.

The first activity value is measured, for example, in the following manner. First, the measurement sample prepared in the above manner is brought into contact with the ATP competitive inhibitor for Src. The contact is usually performed in a measurement sample solution. Then, the measurement sample brought into contact with the inhibitor is brought into contact with a substrate of tyrosine kinase and the phosphorylated substrate is detected to measure the first activity value.

The final concentration of the Src inhibitor during contact with the measurement sample may be suitably set according to the type of the inhibitor, the concentration of the substrate of tyrosine kinase, and the concentration of the phosphate group donor to be described later (e.g. ATP). For example, it may be set to a range of about 0.1 to 10 times based on the final concentration of ATP as the phosphate group donor.

The second activity value can be measured in the same manner as the first activity value except that the measurement sample is not brought into contact with the Src inhibitor.

The measurement of the first and second activity values is preferably performed by mixing the measurement sample, the substrate, and the phosphate group donor and detecting the substrate phosphorylated by the activity of tyrosine kinase. The phosphate group of the phosphate group donor is incorporated into the substrate by a tyrosine kinase-mediated reaction so that the activity of tyrosine kinase can be measured by detecting the phosphorylated substrate.

The substrate for tyrosine kinase is preferably a substrate having low specificity to the type of tyrosine kinase (hereinafter referred to as a "universal substrate") or a substrate having high specificity to a certain tyrosine kinase.

The substrate having high specificity to certain tyrosine kinases, for example to EGFR, includes Grb2, myelin basic protein (MBP), histone H2B (HH2B), phospholipase C gamma and the like. The substrate having high specificity to EGFR may be a commercially available substrate such as GST-EGFR substrate (Stratagene). The GST-EGFR substrate is a fusion protein of glutathione-S-transferase (GST) and a substrate synthesized so as to be phosphorylated by the enzyme activity of EGFR.

The above universal substrate is the substrate which has low specificity to the type of tyrosine kinase, i.e. the substrate for multiple types of tyrosine kinases. Thus, the universal substrate is preferably a known synthetic peptide which is synthesized so as to have low specificity to the type of tyrosine kinases. A specifically preferable example of such a synthetic peptide is a peptide having an amino acid sequence comprising a glutamic acid residue and a tyrosine residue. Such a synthetic peptide may be, for example, a commercially available synthetic peptide such as Poly-Glu4-Tyr (biotin conjugate; UPSTATE). Other examples of the synthetic peptide include the synthetic peptides described as substrates for tyrosine kinase in such references as Norio Sasaki et al., The Journal of Biological Chemistry, Vol. 260, pages 9793-9804 (1985), Sergei Braun et al., The Journal of Biological Chemistry, Vol. 259, pages 2051-2054 (1984), and M. Abdel-Ghany et al., Proceeding of The National Academy of Science, Vol. 87, pages 7061-7065 (1990). The synthetic peptides disclosed in these references are consisting of amino acid sequences comprising a glutamic acid residue (Glu) and a tyrosine residue (Tyr) and synthesized so that Tyr is phosphorylated by two or more tyrosine kinases.

Specific examples of the amino acid sequences of the synthetic peptides include the followings:

- an amino acid sequence in which a sequence consisting of four Glu's and one Tyr is repeated two times or more (hereinafter referred to as "amino acid sequence a");
- an amino acid sequence in which a sequence consisting of one Glu and one Tyr is repeated two times or more (hereinafter referred to as "amino acid sequence b");
- an amino acid sequence in which a sequence consisting of six Glu's, one Tyr and three alanine residues (Ala) is repeated two times or more (hereinafter referred to as "amino acid sequence c");
- an amino acid sequence in which a sequence consisting of one Glu, one Tyr, and one Ala is repeated two times or more (hereinafter referred to as "amino acid sequence d");
- an amino acid sequence in which a sequence consisting of two Glu's, one Tyr, six Ala's, and five lysine residues (Lys) is repeated two times or more (hereinafter referred to as "amino acid sequence e").

The reference (Tony Hunter, The Journal of Biological Chemistry, Vol. 257, pages 4843-4848 (1982)) reports that an acidic amino acid residue is important for the phosphorylation of Tyr by tyrosine kinase. Thus, the substrate is preferably the amino acid sequences a and c which contain more Glu's, acidic amino acid residue.

Examples of the phosphate group donor include adenosine triphosphate (ATP), adenosine 5'-O-(3-thiotriphosphate) (ATP-$\gamma$S), 32 P-labeled adenosine 5'-O-(3-triphosphate) ($\gamma$-[32 P]-ATP), adenosine diphosphate (ADP), and adenosine monophosphate (AMP).

The detection of the phosphorylated substrate is preferably carried out by separating the phosphorylated substrate from other proteins and detecting the substrate.

In order to separate the phosphorylated substrate from other proteins, the substrate of tyrosine kinase may have an affinity tag. The use of the substrate having the affinity tag and a solid phase having a substance capable of binding to the affinity tag (hereinafter referred to as a "binding substance") makes it possible to easily separate the phosphorylated substrate from other proteins and collect it. Specifically, the phosphorylated substrate can be collected by collecting a complex of the phosphorylated substrate and the solid phase and dissociating the link between the affinity tag and the binding substance of the solid phase in the complex.

The affinity tag is not specifically limited so long as it has a corresponding binding substance and it does not prevent the binding between the substrate and tyrosine kinase or phosphorylation of the substrate. As the affinity tag, for example, a polypeptide and hapten may be used. Specifically, GST, histidine, maltose binding protein, FLAG peptide (Sigma), Myc tag, hemagglutinin (HA) tag, Strep tag (IBA GmbH), biotin, avidin, streptavidin and the like may be used.

The substrate having the affinity tag may be the one obtained by chemically linking the affinity tag and the substrate. Alternatively, when the affinity tag is a polypeptide, the one obtained by introducing a vector having a recombinant gene encoding a fusion protein of the affinity tag and the substrate into a host and collecting the fusion protein produced by the host may also be used.

The binding substance corresponding to the affinity tag is not specifically limited so long as it can reversibly bind to the affinity tag. Examples of the binding substance include glutathione, nickel, amylose, anti-FLAG antibody (Sigma), anti-Myc antibody, anti-HA antibody, and Strep-Tactin (IBA GmbH).

The solid phase is not specifically limited so long as it is a support that can bind to the binging substance. Examples of the material for the solid phase include polysaccharides, plastics, and glass. Examples of the shape of the solid phase include beads and gel. Specific examples of the solid phase include Sepharose beads, agarose beads, magnetic beads, glass beads, and silicone gel. These sold phases may be charged in columns for use.

The combinations of the affinity tag and the solid phase having the binding substance include the following examples.

When the affinity tag is GST, the solid phase used may be glutathione Sepharose beads (hereinafter referred to as "glutathione beads"). The measurement of tyrosine kinase activity using this combination can be specifically carried out as follows. The cell membrane fraction having contacted with the tyrosine kinase activity inhibitor is brought into contact with the substrate linked to GST. By adding glutathione beads thereto, glutathione beads to which the phosphorylated substrate has been bound are obtained. After collecting these glutathione beads, reduced glutathione is added to dissociate the link between GST and glutathione beads and the substrate is collected.

Alternatively, the substrate linked to GST is first brought into contact with glutathione beads to obtain complexes of the substrate and the beads. Then, the cell membrane fraction having contacted with the tyrosine kinase activity inhibitor is brought into contact with the complexes and is collected. Reduced glutathione is added thereto to dissociate the link between GIST and glutathione beads and the substrate is collected.

When the affinity tag is histidine, the solid phase having the binding substance can be nickel agarose beads, for example. The link between histidine and nickel can be dissociated with acid such as glycine-HCl or imidazole, for example.

When the affinity tag is maltose binding protein, the solid phase having the binding substance can be amylose magnetic beads, for example. The link between maltose binding protein and amylose can be dissociated with free amylose, for example.

When the affinity tag is FLAG peptide, the solid phase having the binding substance can be FLAG affinity gel (Sigma), for example. The link between FLAG peptide and FLAG affinity gel can be dissociated with acid such as glycine-HCl or 3×FLAG peptide (Sigma), for example.

When the affinity tag is Myc tag, the solid phase having the binding substance can be agarose beads bound to anti-Myc antibody.

When the affinity tag is HA tag, the solid phase having the binding substance can be agarose beads bound to anti-HA antibody.

The links between Myc tag and anti-Myc antibody and between HA tag and anti-HA antibody can be dissociated by denaturing proteins by adding acid or alkaline, for example. In these cases, the acid or alkaline to be used is preferably the one which allows the recovery of non-denatured protein. Specifically, the acid includes hydrochloric acid and alkaline includes sodium hydroxide.

When the affinity tag is Strep tag, the solid phase having the binding substance can be Strep-Tactin immobilized gel column (IBA GmbH), for example. The link between Strep tag and Strep-Tactin can be dissociated with desthiobiotin which reversibly react with streptavidin, for example.

After the contact of tyrosine kinase with the substrate, the enzyme reaction may be terminated by heating, cooling or addition of an enzyme inhibitor such as EDTA before collecting the phosphorylated substrate. By terminating the enzyme reaction, variation in measurement results from sample to sample can be prevented due to the progression of the enzyme reaction during collection of the substrate.

It is preferable to measure the phosphorylated substrate by attaching a labeling substance to the phosphorylated

substrate separated from the solid phase as described above and detecting the labeling substance. The labeling substance includes, but not limited to, for example, fluorescent substances, enzymes, and radioisotopes. Examples of the fluorescent substances include fluorescein, coumarin, eosin, phenanthroline, pyrene, rhodamine, Cy3, Cy5, FITC, and Alexa Fluor (registered trademark). Examples of the enzymes include alkaline phosphatase and peroxidase. Examples of the radioisotopes include 32 P, 33 P, 131 I, 125 I, 3 H, 14 C, and 35 S.

When the labeling substance is the enzyme, the detection may be carried out by detecting the coloring derived from the reaction of the enzyme with its substrate. When the enzyme is an alkaline phosphatase, the substrate includes a mixture of nitrotetrazolium blue chloride (NBT) and 5-bromo-4-chloro-3-indoxyl phosphate (BCIP). When the enzyme is peroxidase, the substrate includes diaminobenzidine (DAB).

The linkage between the phosphorylated substrate and the labeling substance may be performed in a known manner in the art. For example, the phosphorylated substrate can be linked to the labeling substance by using an antibody which has the labeling substance and can specifically bind to the phosphorylated substrate (hereinafter referred to as a "phosphorylated substrate recognizing antibody").

The linkage between the phosphorylated substrate and the labeling substance may also be performed by using the phosphorylated substrate recognizing antibody and an antibody which is capable of binding to the phosphorylated substrate recognizing antibody and has the labeling substance (hereinafter referred to as a "secondary antibody"). In this case, the labeling substance can substantially be linked to the phosphorylated substrate via the phosphorylated substrate recognizing antibody and the secondary antibody.

The linkage between the phosphorylated substrate and the labeling substance may also be performed by using the phosphorylated substrate recognizing antibody, the secondary antibody having biotin, and avidin having the labeling substance. In this case, the labeling substance can substantially be linked to the phosphorylated substrate via the phosphorylated substrate recognizing antibody, the secondary antibody, biotin, and avidin. The secondary antibody may have avidin and biotin may have the labeling substance.

The linkage between the phosphorylated substrate and the labeling substance may also be performed by using the phosphorylated substrate recognizing antibody having biotin and avidin having the labeling substance, or using the phosphorylated substrate recognizing antibody having avidin and biotin having the labeling substance.

When the labeling substance as above is used, the phosphorylated substrate can be detected by detecting the signal generated from the labeling substance, thereby allowing tyrosine kinase activity to be measured.

The phosphorylated substrate recognizing antibody and secondary antibody may be antibodies obtained by a conventionally-known method. Examples of such a method include a method for obtaining antibodies from blood of an animal immunized with an antigen and a method for obtaining antibodies by gene recombination. The antibodies may be polyclonal or monoclonal and may be a fragment of antibodies or a derivative thereof. Two or more of the antibodies may be mixed for use. Examples of the fragment and derivative of antibodies include Fab fragment, E"(ab') fragment, F(ab) 2 fragment, and sFv fragment (Blazar et al., Journal of Immunology, Vol.159: pages 5821-5833 (1997) and Bird et al., Science, vol. 242 : pages 423-426 (1988)). The antibody may belong to any class such as IgG and IgM.

The detection method of the phosphorylated substrate may be appropriately selected according to the type of the labeling substance. For example, when the labeling substance is the fluorescent substance or enzyme, the phosphorylated substrate can be detected by Western blotting. The phosphorylated substrate can be detected by blotting the phosphorylated substrate separated by electrophoresis such as SDS-PAGE to a membrane, incubating the membrane in a buffer containing the phosphorylated substrate recognizing antibody to allow for the binding between the antibody and the phosphorylated substrate, allowing the secondary antibody having the labeling substance to bind to the phosphorylated substrate recognizing antibody, and detecting the labeling substance. When the phosphorylated substrate has the affinity tag, the phosphorylated substrate can be measured in a similar manner to the case of using Western blotting by separating the phosphorylated substrate using the affinity tag and carrying out slot blotting instead of Western blotting.

When the labeling substance is the fluorescent substance, the phosphorylated substrate can also be detected by placing a solution containing the phosphorylated substrate in a tube, adding the phosphorylated substrate recognizing antibody having the fluorescent substance in order to allow for the binding of the antibody with the phosphorylated substance, and measuring the fluorescent intensity.

When the labeling substance is the enzyme, the phosphorylated substrate can be detected by enzyme linked immunosorbent assay (ELISA). ELISA includes both direct adsorption and sandwich methods.

The direct adsorption method is a method including the direct adsorption of the phosphorylated substrate to the solid phase. The method may include, for example, adsorbing the phosphorylated substrate on the surface of the solid phase, allowing for the binding between the phosphorylated substrate recognizing antibody having the enzyme and the phosphorylated substrate, developing color by using the substrate to the enzyme of the phosphorylated substrate recognizing antibody, and detecting the generated color.

The sandwich method is a method including detecting the phosphorylated substrate by using two phosphorylated substrate recognizing antibodies which recognize two different sites of the phosphorylated substrate. The method may

include, for example, allowing the phosphorylated substrate recognizing antibody (hereinafter referred to as a "solid phase antibody") to bind to the solid phase, allowing the phosphorylated substrate to bind to the sold phase antibody, allowing for the binding of the phosphorylated substrate recognizing antibody having the enzyme (hereinafter referred to as a "labeling antibody") to the phosphorylated substrate, and detecting color generated by the reaction between the enzyme of the labeling antibody and the substrate to the enzyme.

When the labeling substance is the radioisotope, the phosphorylated substrate can be detected by radioimmunoassay (RIA). Specifically, the phosphorylated substrate recognizing antibody having the radioisotope is linked to the phosphorylated substrate and radiation is measured by a scintillation counter.

As described above, the first activity value is a kinase activity value measured in the presence of the Src inhibitor and the second activity value is a kinase activity value measured in the absence of the inhibitor, and thus the first activity value is usually predicted to be lower than the second activity value. However, the first activity value may be the same as or higher than the second activity value.

Therefore, in the case of using, for example, the value obtained by subtracting the first activity value from the second activity value as the value of the difference between the first and second activity values in the evaluation of the inhibitory capacity of the Src inhibitor, when the value of the difference is low (including zero and a negative value), the inhibitory capacity of the Src inhibitor can be evaluated to be low. On the other hand, when the value obtained by subtracting the first activity value from the second activity value is high, the inhibitory capacity can be evaluated to be high.

In the case of evaluating the inhibitory capacity of the Src inhibitor using, for example, the value obtained by dividing the first activity value by the second activity value as the ratio of the first and second activity values, when the ratio is high, the inhibitory capacity can be evaluated to be low. On the other hand, when the value obtained by dividing the first activity value by the second activity value is low, the inhibitory capacity can be evaluated to be high. Further, in the case of evaluating the inhibitory capacity of the inhibitor using the inhibition rate of the Src inhibitor in the measurement sample, i.e., the value of the equation: "100-{(the first activity value) / (the second activity value) }x100", when the value is low (including zero and a negative value), the inhibitory capacity can be evaluated to be low. On the other hand, when the inhibition rate is high, the inhibitory capacity can be evaluated to be high.

The evaluation of the inhibitory capacity of the Src inhibitor can be experientially performed by accumulating data of the first and second activity values regarding the sensitivity and insensitivity of tumor cells to the ATP competitive inhibitor for EGFR/HER2. From the viewpoint of more accurate evaluation, it is preferable that the inhibitory capacity of the Src inhibitor in the measurement sample is evaluated based on the results obtained by comparing the difference or ratio of the first activity value and the second activity value with a threshold value to be described later.

That is, in the case of using, for example, the value obtained by subtracting the first activity value from the second activity value as the value of the difference between the first and second activity values in the above-described evaluation, when the value of the difference is compared with the threshold value and the value of the difference is lower than the threshold value, the inhibitory capacity of the Src inhibitor can be evaluated to be low. On the other hand, when the value obtained by subtracting the first activity value from the second activity value is compared with the threshold value and the value of the difference is more than the threshold value, the inhibitory capacity can be evaluated to be high.

In the case of evaluating the inhibitory capacity of the Src inhibitor using, for example, the value obtained by dividing the first activity value by the second activity value as the ratio of the first and second activity values, when the ratio is compared with the threshold value and the ratio is higher than the threshold value, the inhibitory capacity can be evaluated to be low. On the other hand, when the value obtained by dividing the first activity value by the second activity value is compared with the threshold value and the ratio is less or equal to the threshold value, the inhibitory capacity can be evaluated to be high. In the case of evaluating the inhibitory capacity of the inhibitor using the inhibition rate of the Src inhibitor in the measurement sample, i.e., the value of the equation: "100-{(the first activity value) /(the second activity value)} x100", when the inhibition rate is compared with the threshold value and the inhibition rate is lower than the threshold value, the inhibitory capacity can be evaluated to be low. On the other hand, when the inhibition rate is compared with the threshold value and the inhibition rate is more than the threshold value, the inhibitory capacity can be evaluated to be high.

The threshold value may be suitably set according to the type of the Src inhibitor and the type of tumor cells. The threshold value can be set, for example, based on the first and second activity values which are acquired by using tumor cells which are known to be sensitive to the ATP competitive inhibitor for EGFR/HER2 and a specific Src inhibitor in the same manner as the above measurement of tyrosine kinase activity. When the threshold value is calculated as, for example, the inhibition rate of the Src inhibitor, the threshold value can be set in a range of usually 0 to 80%, preferably 10 to 70%, more preferably 20 to 50%.

The determination method of the present embodiment determines that tumor cells in a biological sample have sensitivity to the ATP competitive inhibitor for EGFR/HER2 when evaluating that the inhibitory capacity of the ATP competitive inhibitor for Src is low in the above-described evaluation, and/or the method determines that the tumor cells have no sensitivity to the ATP competitive inhibitor for EGFR/HER2 when evaluating that the inhibitory capacity of the ATP competitive inhibitor for Src is high.

The method for determining sensitivity in the present embodiment is not limited to the above-described description. The method for determining sensitivity in the present embodiment may determine that tumor cells in the biological sample have high sensitivity to the ATP competitive inhibitor for EGFR/HER2, for example, when evaluating that the inhibitory capacity of the ATP competitive inhibitor for Src is low and/or the method may determine that the tumor cells have low sensitivity to the ATP competitive inhibitor for EGFR/HER2 when evaluating that the inhibitory capacity of the ATP competitive inhibitor for Src is high.

In the present specification, the levels of sensitivity of the tumor cells to the ATP competitive inhibitor for EGFR/HER2 in the determination results may have the same meaning as the presence or absence of the sensitivity to the inhibitor.

The computer program of the present embodiment will be described below.

The computer program of the present embodiment is a computer program for allowing a computer to realize a method for determining sensitivity of tumor cells to inhibitors of EGFR and HER2. Fig. 1 is a block diagram showing a hardware configuration of a system for determining sensitivity of EGFR/HER2 inhibitor which includes a determination device 100 which executes the program of the present embodiment and a measurement device 200 for kinase activity in a measurement sample.

The system for determining sensitivity includes the determination device 100 and the measurement device 200 for kinase activity value which are connected by a cable 300. Data of the value of kinase activity which is measured by the measurement device 200 is sent to the determination device 100 via the cable 300. The determination device 100 is a device for analyzing the data output from the measurement device 200, determining sensitivity of tumor cells to EGFR/HER2 inhibitor, and outputting determination results. The determination device 100 and the measurement device 200 are configured as an integrated device.

The determination device 100 is mainly configured by a main body 110, a display unit 120, and an input device 130. In the main body 110, the CPU 110a, a ROM 110b, a RAM 110c, a hard disk 110d, a read-out device 110e, and an input/output interface 110f, and an image output interface 110g are data-communicably connected by a bus 110h.

The CPU 110a can execute computer programs stored in the ROM 110b and the computer programs loaded in the RAM 110c. The ROM 110b is configured by mask ROM, PROM, EPROM, EEPROM, and the like, and is recorded with computer programs to be executed by the CPU 110a, data used for the same.

The RAM 110c is configured by SRAM, DRAM, and the like. The RAM 110c is used to read out the computer programs recorded on the ROM 110b and the hard disc 110d. The RAM 110c is used as a work region when the CPU 110a executes these computer programs.

The hard disc 110d is installed with various computer programs to be executed by the CPU 110a such as operating system and application system program, as well as data used in executing the computer program. In order to allow the determination device 100 to realize the determination method of the present embodiment to be described later, the computer program 140a and a threshold value to be used for evaluation of the inhibitory capacity of the Src inhibitor are installed on the hard disk 110d.

The read-out device 110e is configured by a flexible disk drive, a CD-ROM drive or a DVD-ROM drive. The read-out device 110e is capable of reading out computer programs or data recorded on a portable recording medium 140. An application program 140a for allowing the computer to execute an operation is stored in the portable recording medium 140. It is also possible that the CPU 110a reads out the application program 140a from the portable recording medium 140 and installs the application program 140a on the hard disk 110d.

Operating system providing graphical user interface environment such as Windows (registered trademark) manufactured and sold by US Microsoft Co. is installed in the hard disc 110d.

In the following explanation, the computer program 140a according to the determination of sensibility of EGFR/HER2 inhibitor is configured to operate on the operating system.

The input/output interface 110f includes a serial interface such as USB, IEEE1394, and RS-232C; a parallel interface such as SCSI, IDE, and IEEE1284; and an analog interface such as D/A converter and A/D converter. The input/output interface 110f is connected to the input device 130 including a keyboard and a mouse. Thus, a user can use the input device 130 to input data of tyrosine kinase activity values (first and second activity values) obtained by measuring the measurement sample to the computer main body 110. The measurement device 200 which is capable of measuring tyrosine kinase activity in the measurement sample can be connected to the input/output interface 110f. In this case, the measurement device 200 can input the data of the first and second activity values to the computer main body 110.

The image output interface 110h is connected to the display unit 120 configured by LCD, CRT, or the like, and is configured to output an image signal corresponding to the image data provided from the CPU 110a to the display unit 120. The display unit 120 outputs image data according to the input image signal. The display unit 120 outputs determination results provided from the CPU 110a to be described later.

Fig. 2 is a flow chart showing a process of determining sensitivity of tumor cells to EGFR/HER2 inhibitor by the CPU 110a.

The first and second activity values are measured by bringing the measurement sample prepared from the biological sample containing tumor cells into contact with the substrate in the presence or absence of the Src inhibitor and detecting a signal generated from the phosphorylated substrate in the measurement sample brought into contact with the substrate by the measurement device 200.

The CPU 110a of the determination device 100 acquires the first and second activity values from the measurement device 200 via the input/output interface 110f (step S11) and allows the RAM 110c to store the acquired first and second activity values.

The CPU 110a reads out the first and second activity values stored in the RAM 110c, calculates a value of inhibitory capacity of the Src inhibitor (step S12), and allows the RAM 110c to store the value.

The value of inhibitory capacity of the Src inhibitor is not particularly limited as long as it can evaluate the inhibitory capacity of the ATP competitive inhibitor for the tyrosine kinase Src. Examples thereof include the value of the difference between the first and second activity values, the ratio of the first and second activity values, and the inhibition rate of the Src inhibitor. Examples of the difference of the first and second activity values include a value obtained by subtracting the first activity value from the second activity value and a value obtained by subtracting the second activity value from the first activity value. Examples of the ratio of the first and second activity values include the value obtained by dividing the first activity value by the second activity value and the value obtained by dividing the second activity value by the first activity value. The inhibition rate of the Src inhibitor includes the value (%) of the equation: "100-{(the first activity value)/(the second activity value)} x100".

In the present embodiment, the value (%) of the equation: "100-{(the first activity value)/(the second activity value) } x100" was calculated as the value of inhibitory capacity of the Src inhibitor.

The CPU 110a reads out a threshold value prestored in the hard disk 110d and compares the threshold value with the value of inhibitory capacity of the Src inhibitor (step S13). Here, the threshold value may be set depending on the value of inhibitory capacity of the Src inhibitor and it is not particularly limited. In the present embodiment, the threshold value of the value (%) of the equation: "100-{(the first activity value) / (the second activity value)} x100" was set to 43.4%.

Here, the threshold value has been prestored in the hard disk 110d, however the present invention is not limited thereto. For example, the CPU 110a can receive data of the threshold value input from the input device via the input/output interface 110f. The CPU 110a can also receive data of the threshold value stored in an external storage device via the input/output interface 110f connected to the Internet. Further, the CPU 110a can also receive data of the threshold value recorded on the portable recording medium 140 by reading out it by the read-out device.

The CPU 110a determines that the tumor cells have no sensitivity to EGFR/HER2 inhibitor when the value of inhibitory capacity of the Src inhibitor is more than the threshold value (step S14). The CPU 110a determines that the tumor cells have sensitivity to EGER/HER2 inhibitor when the value of inhibitory capacity of the Src inhibitor is lower than the threshold value (step S15). Here, examples of the value of inhibitory capacity of the Src inhibitor include the value obtained by subtracting the first activity value from the second activity value, the value obtained by dividing the second activity value by the first activity value, and the value (%) of the equation:

"100-{(the first activity value)/(the second activity value)}x100".

The determination in step S14 may be suitably changed according to the value of inhibitory capacity of the Src inhibitor and it should not be limited to the above-described description. For example, when the value of inhibitory capacity of the Src inhibitor is the value obtained by subtracting the second activity value from the first activity value, the value obtained by dividing the first activity value by the second activity value or the value of the equation: "100-{(the first activity value)/(the second activity value) } x100", the CPU 110a can determine that the tumor cells have no sensitivity to EGFR/HER2 inhibitor when the value of inhibitory capacity of the Src inhibitor is lower than the threshold value. The CPU 110a can determine that the tumor cells have sensitivity to EGFR/HER2 inhibitor when the value of inhibitory capacity of the Src inhibitor is more than the threshold value.

The CPU 110a stores the determination results in the hard disk 110d and outputs them on the display unit 120 via the image output interface 110g (step S16).

In this case, the CPU 110a outputs only the determination result, however it may output instruction as to whether to provide treatment with EGFR/HER2 inhibitor to a cancer patient in whom the biological sample containing tumor cells is collected. That is, the CPU 110a outputs the determination result and instruction not to provide treatment with EGFR/HER2 inhibitor to the display unit 120 when determining that the tumor cells have no sensitivity to EGFR/HER2 inhibitor. On the other hand, when determining that the tumor cells have sensitivity to EGFR/HER2 inhibitor, the CPU 110a may output the determination result and instruction for providing treatment with EGFR/HER2 inhibitor to the display

unit 120

EXAMPLE

In the following examples, the present invention will be described more details, however, it is not intended that the present invention is limited to the following embodiments.

Breast cancer cell lines used in this Example and the culture conditions thereof were shown in Table 3. Penicillin (100 units/ml), Streptomycin (100 μg/ml), and Amphotericin B (0.25 μg/ml) are contained in all media shown in the culture conditions. All of these cell lines can be purchased from American Type Culture Collection (ATCC). Further, basal medium, fetal bovine serum (FBS), bovine insulin, glutathione, and human EGF which are used to culture the cell lines are marketed and commonly-available.

[Table 3]

| Cell line | ATCC No. | Basal medium | FBB concentration (%) | Addition to medium | Culture conditions |
|---|---|---|---|---|---|
| AU565 | CRL-2351 | RPMI 1640 | 10 | | 37°C. 5% $CO_2$ |
| BT-20 | HTB-19 | MEM | 10 | | 37°C, 5% $CO_2$ |
| BT-474 | HTB-20 | DMEM | 10 | | 37°C, 5% $CO_2$ |
| BT-483 | HTB-121 | RPMI 1640 | 20 | 10 μg/ml Bovine insulin | 37°C, 5% $CO_2$ |
| BT-549 | HTB-122 | RPMI 1640 | 10 | 1 μg/ml Bovine insulin | 37°C, 5% $CO_2$ |
| CAMA-I | HTB-21 | MEM | 10 | | 37°C, 5% $CO_2$ |
| HCC202 | CRL-2316 | RPMI 1640 | 10 | | 37°C, 5% $CO_2$ |
| HCC1419 | CRL-2326 | RPMI 1640 | 10 | | 37°C, 5% $CO_2$ |
| HCC1428 | CRL-2327 | RPMI 1640 | 10 | | 37°C, 5% $CO_2$ |
| HCC1569 | CRL-2330 | RPMI 1640 | 10 | | 37°C, 5% $CO_2$ |
| HCC1806 | CRL-2335 | RPMI 1640 | 10 | | 37°C, 5% $CO_2$ |
| HCC1937 | CRL-2336 | RPMI 1640 | 10 | | 37°C, 5% $CO_2$ |
| HCC1954 | CRL-2338 | RPMI 1640 | 10 | | 37°C, 5% $CO_2$ |
| HS578T | HTB-126 | DMEM | 10 | 10 μg/ml Bovine insulin | 37°C, 5% $CO_2$ |
| MCF7 | HTB-22 | MEM | 10 | 10 μg/ml Bovine insulin | 37°C, 5% $CO_2$ |
| MDA-MB-157 | HTB-24 | Leibovitz'sL-15 | 10 | | 37°C 100% air |
| MDA-MB-231 | HTB-26 | Leibovitz's L-15 | 10 | | 37°C 100% air |
| MDA-MB-415 | HTB-128 | Leibovitz's L-15 | 15 | 10 μg/ml Bovine insulin, 10 μg/ml Glutathione | 37°C 100% air |
| MDA-MB-435S | HTB-129 | Leibovitz'sL-15 | 10 | 10 μg/ml Bovine insulin | 37°C 100% air |
| MDA-MB-436 | HTB-130 | Leibovitz'sL-15 | 10 | 10 μg/ml Bovine insulin, 10 μg/ml Clutathione | 37°C 100% air |
| MDA-MB-453 | HTB-131 | leibovitz's L-15 | 10 | | 37°C 100% air |
| MDA-MB-468 | HTB-132 | Leibovitz's L-15 | 10 | | 37°C 100% air |

(continued)

| Cell line | ATCC No. | Basal medium | FBB concentration (%) | Addition to medium | Culture conditions |
|---|---|---|---|---|---|
| SK-BR-3 | HTB-30 | McCoy's 5a | 10 | | 37°C, 5% $CO_2$ |
| T-47D | HTB-133 | RPMI 1640 | 10 | 10 μg/ml Bovine insulin | 37°C, 5% $CO_2$ |
| UACC-812 | CRL-1897 | Leibovitz'sL-15 | 20 | 20 ng/ml Human EGF | 37°C 100% air |
| UACC-893 | CRL-1902 | Leibovitz's L-15 | 10 | | 37°C 100% air |
| ZR-75-1 | CRL-1500 | | 10 | | 37°C. 5% $CO_2$ |
| ZR-75-30 | CRL-1504 | RPMI 1640 | 10 | | 37°C, 5% $CO_2$ |

The sensitivity of cell lines to EGFR/HER2 inhibitor was examined by culturing breast cancer cell lines shown in Table 3 in the presence of EGFR/HER2 inhibitor and examining growth inhibition effects on the cell lines.

The breast cancer cell lines shown in Table 3 were seeded in a 96 well assay plate (# 3917: Costar) at 4,000 cells/well, and cultured at 37°C. After 24 hours, the medium was changed to a medium containing a DMSO solution of lapatinib ditosylate hydrate (hereafter also referred to as lapatinib; #L-4804: LC Laboratories) with various concentrations, and the culture was continued at 37°C for 72 hours. The concentrations of lapatinib in the medium were 0 (control), 0.1, 1, 10, 30, 100, 300, 1,000 and 3,000, and 10,000 nM. The concentration of DMSO in the medium is constant (0.1(v/v) %) in all of the wells. The cell number in the wells at 0 and 72 hours after exchange of the medium was measured using Cell Titer-Glo Luminescent Cell Viability Assay (#G7571: Promega KK.). The lapatinib concentration to yield half the cell number of the control in 72 hours was determined by fitting with a logistic curve and the concentration was IC50. The cell line whose IC50 was less than 1 μM was defined as a "sensitive line", while the cell line whose IC50 was 1 μM or more was defined as an "insensitive line". The results are shown in Tables 4 and 5.

[Table 4]

| Sensitive line | $IC_{50}$ (μM) |
|---|---|
| BT-474 | 0.080 |
| HCC1419 | 0.124 |
| AU565 | 0.157 |
| ZR-75-30 | 0.211 |
| MDA-MB-453 | 0.309 |
| SK-BR-3 | 0.315 |
| UACC-893 | 0.482 |
| UACC-812 | 0.490 |

[Table 5]

| Insensitive line | $IC_{50}$(μM) |
|---|---|
| HCC 1954 | 1.468 |
| HCC202 | 2.470 |
| MDA-MB-468 | 3.782 |
| HCC1569 | 4.815 |
| BT-20 | >10 |
| BT-483 | >10 |

(continued)

| Insensitive line | $IC_{50}$(μM) |
|---|---|
| BT-549 | >10 |
| CAMA-1 | >10 |
| HCC1428 | >10 |
| HCC1806 | >10 |
| HCC1937 | >10 |
| HS578T | >10 |
| MCF7 | >10 |
| MDA-MB-157 | >10 |
| MDA-MB-231 | >10 |
| MDA-MB-415 | >10 |
| MDA-MB-435S | >10 |
| MDA-MB-436 | >10 |
| T-47D | >10 |
| ZR-75-1 | >10 |

(Example 1)

(1) Cell culture

Breast cancer cell lines shown in Table 3 were cultured in a 150 mm dish so as to be about 80% confluent, the cells were recovered using PBS and a scraper, and a cell pellet was obtained by centrifugal operation (900xg, for 5 minutes, at 4°C) . The pellet was frozen with liquid nitrogen and stored at -80°C until preparation of the measurement sample.

(2) Preparation of measurement sample for tyrosine kinase activity

500 μl of NP40(-) solubilized buffer (20 mM HEPES (pH7.4)), 10 (w/v) % glycerol, 0.2% protease inhibitor cocktail (#P8340: Sigma-Aldrich (product composition: 104 mM AEBSF, 80 μM aprotinin, 4 mM bestatin, 1.4 mM E-64, 2 mM leupeptin, 1.5 mM Pepstatin A), 50 mM NaF and 200 μM Na3VO4) was added to the cell pellet, followed by stirring for 2 seconds three times using a vortex mixer. After being allowed to stand for 10 minutes, the soluble fraction was removed by centrifugal operation (at 20000xg, for 30 minutes, at 4°C). The obtained pellet was resuspended in 500 μl of NP40 (-) solubilized buffer, the soluble fraction was again removed by centrifugal operation (at 20000xg, for 20 minutes, at 4°C), and the pellet was washed. The pellet was resuspended by adding 500 μl of NP40 (+) solubilized buffer (a buffer prepared by adding NP-40 to the NP40(-) solubilized buffer at a final concentration of 1%) to the pellet, pipetting fifty times, and vortexing for 2 seconds three times. After being allowed to stand for 10 minutes, a cell membrane fraction containing a membrane protein was obtained by centrifugal operation (at 20000xg, for 40 minutes, at 4°C). The protein concentration of the fraction was measured by DC protein assay (#500-0116 JA: Bio-Rad) and the fraction was used as a sample for kinase activity measurement. The sample was frozen with liquid nitrogen and stored at -80°C until used for the following kinase activity measurement.

(3) Evaluation of inhibitory capacity of ATP competitive inhibitor for Src in measurement sample

NeutrAvidin Coated Plates (HBC) (96 white plate; #15509: Pierce) were washed three times with 300 μl of TBS-T (20 mM Tris-HCl (pH 7.4)), 150 mM NaCl, 0.1% Tween), and then 100 μl of Poly-Glu4-Tyr biotin conjugate (#12440: Upstate) which had been diluted to 1 μg/ml with TBS (20 mM Tris-HCl (pH 7.4), 150 mM NaCl) was added to the wells. After shaking at 37°C and 400 rpm for 90 minutes, the wells were washed with 300 μl of TBS-T three times and a measuring plate was obtained.

The measurement sample was diluted with a kinase buffer (20 mM HEPES (pH 7.4), 25 mM MgCl2, 1.25 mM

MnCl2, 250 μM Na3VO4, 12.5 (w/v) % glycerol) so as to adjust the protein concentration to 6.25 μg/ml. The diluted measurement sample was transferred to wells of a 96 well V bottom plate. A DMSO solution (inhibitor concentration: 5mM) containing one type of tyrosine kinase inhibitors (Inhibitors 1 to 10 shown in Table 6) was added to the wells at a ratio of 1:160 based on the diluted measurement sample solution, followed by mixing thereof. As the control, DMSO was added to the diluted measurement sample at the same ratio as the solution of the inhibitor. The used tyrosine kinase inhibitors are represented as Inhibitors 1 to 10 in Table 6.

[Table 6]

| | Name | Manufacturer | CAS No. |
|---|---|---|---|
| Inhibitor 1 | EGFR Inhibitor (AG1478) | Calbiochem Corp. | 175178-82-2 |
| Inhibitor 2 | EGFR:Inhibitor II(BIBX1382) | Calbiochem Corp. | 196612-93-8 |
| Inhibitor 3 | EGFR/HER2 Inhibitor (4457W) | Calbiochem Corp. | 179248-61-4 |
| Inhibitor 4 | EGFR/HER2 Inhibitor (GW2974) | Sigma-Aldrich Corp. | 202272-68-2 |
| Inhibitor 5 | EGFR/HER2 Inhibitor (GW583340Dihydrochloride) | Sigma-Aldrich Corp. | 388082-81-3 |
| Inhibitor 6 | PDGFR Inhibitor IV | Calbiochem Corp. | 627518-40-5 |
| Inhibitor 7 | $VEGFR_{Inhibitor\ III}$(KRN633) | Calbiochem Corp. | 286370-15-8 |
| Inhibitor 8 | VEGFR2 Inhibitor (ZM323881 Hydrochloride) | Tocris Bioscience Corp | 324077-30-7 |
| Inhibitor 9 | Met Inhibitor | Calbiochem Corp. | 658084-23-2 |
| Inhibitor 10 | Src Inhibitor 1 | Calbiochem Corp. | 179248-59-0 |

After addition of the inhibitors, a mixed solution of the measurement sample and the inhibitor was allowed to stand at room temperature for 10 minutes. 40 μl of the mixed solution was transferred to wells of the measuring plate and 10 μl of an ATP solution (20 mM HEPES (pH 7.4), 125 mM ATP) as the phosphate group donor was added to the wells.

The composition of the solution in the wells is as follows: protein concentration: 5μg/ml, 20 mM HEPES (pH7.4), 20 mM MgCl2, 1 mM MnCl2, 200 μM Na3VO4, 10 (w/v) % glycerol, 25 μM ATP, 25 μM kinase inhibitor.

After shaking the measuring plate at 37°C and 400 rpm for 60 minutes, the solution was removed from the measuring plate, and the wells were washed with 300 μl of TBS-T three times. 100 μl of Starting Block T20 (TBS) (#37543: Pierce) as a blocking solution was added to the wells, followed by blocking at 37°C and 400 rpm for 10 minutes. The blocking solution was removed from the measuring plate. 100 μl of a solution prepared by diluting HRP-labeled anti-phosphorylated tyrosine antibody (#sc-508 HRP: Santa Cruz) with Starting Block T20 (TBS) at a ratio of 1 : 1000 was added to the wells, followed by shaking at 37°C and 400 rpm for 90 minutes. Then, the wells were washed with 300 μl of TBS-T five times. 100 μl of SuperSignal ELISA Pico Chemiluminescent Substrate (#37070: Pierce) as a substrate was added to the wells, followed by shaking at room temperature for 1 minute, Thereafter, the emission from the wells was measured by a GENios microplate reader (manufactured by Tecan Trading AG). The inhibitory capacity of the kinase inhibitors in the measurement sample was evaluated by an inhibition rate (value of "100-{ (measurement value of activity in the presence of inhibitors) / (measurement value of activity in the absence of inhibitors)}x100") calculated base on a ratio of the measurement value of tyrosine kinase activity in the presence of the inhibitors and the measurement value of the control (in the absence of the inhibitors). The calculated inhibition rates are shown in Table 7. Based on the calculated inhibition rate, graphs showing the inhibition rates of the tyrosine kinase inhibitors in the lapatinib-sensitive cell line group and the lapatinib-insensitive cell line group which are classified in Reference example 1 were created. The graphs are shown in Fig. 3. In Fig. 3, p represents a significant probability.

[Table 7]

| Cell line | Inhibition rate (%) | | | | | | | | | |
|---|---|---|---|---|---|---|---|---|---|---|
| | Inhibito r1 | Inhibitor 2 | Inhibitor 3 | Inhibitor 4 | Inhibitor 5 | -Inhibitor 6 | Inhibitor 7 | Inhibitor 8 | Inhibitor 9 | Inhibitor 10 |
| AU565 | 29.3 | 7.7 | 25.7 | -3.8 | 40.9 | 18.4 | 7.1 | 11.5 | 55.6 | 36.8 |
| BT-20 | 47.2 | 18.9 | 42.2 | 32.4 | 56.3 | 28.1 | 2.3 | 18.5 | 51.4 | 56.6 |
| BT-474 | 26.0 | 10.5 | 15.1 | 24.6 | 42.7 | 30.7 | 30.3 | 18.0 | 54.6 | 37.6 |
| BT-483 | 54.7 | 40.3 | 68.5 | 52.7 | 66.9 | 62.9 | 17.3 | 27.2 | 68.6 | 78.5 |
| BT-549 | 52.9 | 21.7 | 59.8 | 48.8 | 68.8 | 50.1 | 24.0 | 5.1 | 81.4 | 60.6 |
| CAMA-1 | 14.3 | -3.4 | 16.7 | 7.3 | 48.6 | 15.9 | 3.4 | 8.1 | 60.2 | 34.6 |
| HCC202 | 29.7 | 7.7 | 24.5 | 21.3 | 66.2 | 27.1 | 33.4 | 11.9 | 69.1 | 26.3 |
| HCC1419 | 34.9 | 6.2 | 43.3 | 23.8 | 63.6 | 33.5 | 28.4 | 14.7 | 70.7 | 36.3 |
| HCC1428 | 19.3 | 6.8 | 29.8 | 7.9 | 56.1 | 30.0 | 12.9 | 2.2 | 47.5 | 43.7 |
| HCC1569 | 44.7 | 25.0 | 42.3 | 37.9 | 60.7 | 57.8 | 16.6 | 15.5 | 67.0 | 71.3 |
| HCC1806 | 57.9 | 29.6 | 56.4 | 33.7 | 68.4 | 38.8 | 27.7 | 6.4 | 77.3 | 67.1 |
| HCC1937 | 46.0 | 28.1 | 24.1 | 17.9 | 42.3 | 41.8 | 17.3 | 11.1 | 52.6 | 50.1 |
| HCC1954 | 39.4 | 19.7 | 31.8 | 23.2 | 59.5 | 41.5 | 17.6 | 4.1 | 65.7 | 46.5 |
| HS578T | 53.8 | 20.8 | 40.0 | 32.1 | 64.2 | 49.7 | 19.4 | 5.5 | 70.6 | 58.2 |
| MCF7 | 48.0 | 19.9 | 11.3 | 12.1 | 75.4 | 44.4 | 59.1 | 45.7 | 64.3 | 49.2 |
| MDA-MB-157 | 45.0 | 14.3 | 38.0 | 29.2 | 56.1 | 49.0 | 11.2 | 8.3 | 56.8 | 60.3 |
| MDA-MB-231 | 56.4 | 23.5 | 42.0 | 38.9 | 58.2 | 45.7 | 9.7 | 12.8 | 68.0 | 63.6 |
| MDA-MB-415 | 41.3 | 25.0 | 50.5 | 19.6 | 48.1 | 50.9 | 25.1 | 14.0 | 56.7 | 68.6 |
| MDA-MB-435S | 33.4 | 3.8 | 37.2 | 22.6 | 57.8 | 40.6 | 10.5 | 9.8 | 80.3 | 44.9 |
| MDA-MB-436 | 33.9 | 19.0 | 48.7 | 26.3 | 68.5 | 36.1 | 23.1 | 12.1 | 74.0 | 44.9 |
| MDA-MB-453 | 39.8 | 19.9 | 36.9 | 27.9 | 70.4 | 28.9 | 29.4 | 17.7 | 64.4 | 16.3 |
| MDA-MB-468 | 77.4 | 52.1 | 76.9 | 71.6 | 75.4 | 1.4 | 18.5 | 7.1 | 65.0 | 81.0 |
| SK-BR-3 | 46.8 | 18.8 | 44.1 | 31.4 | 75.7 | 45.3 | 45.0 | 25.9 | 66.1 | 43.3 |
| T47D | 29.1 | 7.7 | 30.2 | 27.0 | 50.9 | 37.5 | 22.0 | 38.1 | 42.5 | 42.3 |

(continued)

| | Inhibition rate (%) | | | | | | | | | |
|---|---|---|---|---|---|---|---|---|---|---|
| Cell line | Inhibito r1 | Inhibitor 2 | Inhibitor 3 | Inhibitor 4 | Inhibitor 5 | -Inhibitor 6 | Inhibitor 7 | Inhibitor 8 | Inhibitor 9 | Inhibitor 10 |
| UACC812 | 49.3 | 30.3 | 66.6 | 59.4 | 80.4 | 61.4 | 28.5 | 21.6 | 78.1 | 81.5 |
| UACC893 | 21.2 | 4.1 | 22.9 | -3.1 | 48.4 | 28.0 | 23.9 | 3.3 | 56.9 | 25.6 |
| ZR-75-1 | 20.8 | 9.0 | 38.5 | 7.0 | 44.8 | 32.5 | 18.3 | 0.6 | 53.9 | 55.8 |
| ZR-75-30 | 20.1 | 4.4 | 37.2 | 20.9 | 64.4 | 27.0 | 18.5 | 10.1 | 65.1 | 37.3 |

Lapatinib is an EGFR/HER2 inhibitor. Thus, it was expected that inhibitory effects of an EGFR inhibitor and the EGFR/HER2 inhibitor on tyrosine kinase would become high in the case of the lapatinib sensitive lines classified in Reference example 1. However, such a tendency was not observed in Fig. 3.

On the other hand, a difference in inhibition rate between the lapatinib sensitive line and the lapatinib insensitive line is observed in Inhibitor 10 which is the Src inhibitor. That is, it has been revealed that an inhibitory effect of the Src inhibitor on the kinase activity is low in the case of the lapatinib sensitive line, while the inhibitory effect is high in the case of the lapatinib insensitive line.

ROC analysis when the sensitivity of tumor cells to lapatinib was determined using tyrosine kinase activity inhibitory capacity of Inhibitor 10 as an indicator was performed and an AUC was obtained. The AUC is an indicator showing that the determination with high accuracy can be achieved as the value is close to 1. The AUC obtained by the ROC analysis is shown in Table 8. Further, an optimal threshold value (threshold value (%) as the inhibition rate) was determined from the ROC analysis, the inhibition rate by Inhibitor 10 was compared with the threshold value. When the inhibition rate was lower than the threshold value, it was determined to have lapatinib sensitivity. When the inhibition rate was more than the threshold value, it was determined to have lapatinib insensitivity. The determined sensitivity, specificity, positive predictive value (PPV), and negative predictive value (NPV) of these cases are shown in Table 8.

[Table 8]

| AUC | Threshold value (%) | Sensitivity (%) | Specificity (%) | PPV(%) | NPV(%) |
|---|---|---|---|---|---|
| 0.806 | 43.4 | 88 | 85 | 70 | 94 |

As shown in Table 8, the AUC determined by the determination method of the present embodiment using Inhibitor 10 was very high: 0.806. The sensitivity, specificity, PPV, and NPV were also very high. Therefore, it is found that sensitivity of tumor cells to the ATP competitive inhibitor for EGFR/HER2 can be determined with high accuracy by the determination method of the present embodiment.

Taking into consideration the reference of US 2011027809 which discloses the method for evaluating malignancy in tumor cells and the reference of Konecny et al. which discloses effects of lapatinib on tumor cells, it is suggested that, as an experimental result oppposite that of Example 1, when the inhibitory capacity of the Src inhibitor is evaluated to be high, tumor cells have sensitivity to lapatinib, meanwhile, when the inhibitory capacity of the Src inhibitor is evaluated to be low, tumor cells have insensitivity to lapatinib. More specifically, the reference of US 2011027809 discloses experimental results of tyrosine kinase activity inhibitory effects on 13 types of cell lines caused by the Src inhibitor. Here, Examples of US 2011027809 suggest that, in the case of two cell lines (BT-474 and SK-Br-3) which are lapatinib sensitive lines are disclosed in the reference of Konecny et al., tyrosine kinase activity inhibitory effects caused by the Src inhibitor is high, meanwhile, in the case of three cell lines (MDA-MB231, MCF7, and T47D) which are lapatinib insensitive lines are disclosed in the reference of Konecny et al., tyrosine kinase activity inhibitory effects caused by the Src inhibitor is low.

However, a measurement sample used in Examples of US 2011027809 is treated with $PP_1$ which is a type of Src inhibitor during the preparation thereof, and thus kinase activity of Src in the measurement sample has already been inhibited by PP1. That is, in US 2011027809, the inhibitory activity of Src inhibitor 1 is measured using a measurement sample containing Src whose kinase activity has already been inhibited. Therefore, it is considered that the inhibitory activity of Src inhibitor 1 is measured using an unsuitable material in examples of US 2011027809 and thus the inhibitory activity of Src inhibitor 1 can not be accurately measured.

Therefore, there is neither disclosure nor suggestion for allowing those skilled in the art to arrive at the determination method of the present invention easily in US 2011027809.

(Comparative example 1)

(1) Cell culture

Breast cancer cell lines shown in Table 3 were cultured in a 150 mm dish so as to be about 80% confluent, the cells were recovered using PBS and a scraper, and a cell pellet was obtained by centrifugal operation (900xg, for 5 minutes, at 4°C). The pellet was frozen with liquid nitrogen and stored at -80°C until preparation of the measurement sample.

(2) Preparation of measurement sample for tyrosine kinase activity and confirmation of expression of HER2 protein

An ice-cooled 1% TritonX Lysis buffer (20 mM Tris-HCl (pH 7.5) 150 mM NaCl, 1 mM EDTA, 1% TritonX-100, 20 mM NaF, 1 mM Na3VO4, 0.1% protease inhibitor cocktail (#P8340: Sigma-Aldrich (product composition: 104 mM

AEBSF, 80 μM aprotinin, 4 mM bestatin, 1.4 mM E-64, 2 mM leupeptin, 1.5 5 mM Pepstatin A)) was added to the cell line pellets and the cells were dissolved by pipetting, followed by being allowed to stand on ice for 20 minutes. Insoluble pellets were removed by centrifugal operation (at 20000xg, for 15 minutes, at 4°C) and a lysate was obtained. The protein concentration was measured using DC protein assay (#500-0116JA: Bio-Rad) and the presence of the expression of HER2 was confirmed by Western blotting. Rabbit anti-HER2 polyclonal antibody (#06-562: Upstate) and rabbit anti-GAPDH polyclonal antibody (#2275-pc-100: Trevigen) were respectively used as a primary antibody. Swine anti-rabbit immunoglobulin HRP (#P0399: Dako) was used as a secondary antibody. Detection was carried out using ECL-Plus (#RPN2132: GE Healthcare).

The expression of HER2 protein in the breast cancer cell lines shown in Table 3 was confirmed by Western blotting using the prepared measurement sample. The obtained results are shown in Fig. 4. GAPDH is a control showing that the amount of protein between the samples is almost equal.

The sensitivity of the breast cancer cell lines to lapatinib in Reference example 1, the detection results of HER2 protein in Comparative example 1, and the determination results of sensitivity to lapatinib based on the inhibition rate of the Src inhibitor in Example 1 were shown in Table 9. The mark "+" in the column of HER2 indicates a cell line which is determined to have high HER2 protein expression based on the results of Western blotting, while the mark "-" indicates a cell line which is determined to have low or no HER2 protein expression. In the column of the inhibitory capacity to the Src inhibitor, the term "low" means a cell line whose inhibition rate of the Src inhibitor is lower than the threshold value (=43. 4%) , while the term "high" means a cell line whose inhibition rate of the Src inhibitor is more than the threshold value. In the column of determination, the cell line whose inhibitory capacity to the Src inhibitor was "low" was determined to have sensitivity to lapatinib, while the cell line whose inhibitory capacity to the Src inhibitor was "high" was determined to have insensitivity to lapatinib.

[Table 9]

| | Reference example 1 | comparative example 1 | Example 1 | |
|---|---|---|---|---|
| Cell line | Sensitivity to lapatinib | Expression of HER2 | Inhibitory capacity of Src inhibitor | Determination results |
| BT-474 | Sensitive | + | low | Sensitive |
| HCC1419 | Sensitive | + | low | Sensitive |
| AU565 | Sensitive | + | low | Sensitive |
| ZR-75-30 | Sensitive | + | low | Sensitive |
| MDA-MB-453 | Sensitive | + | low | Sensitive |
| SK-BR-3 | Sensitive | + | low | Sensitive |
| UACC-893 | Sensitive | + | low | Sensitive |
| UACC-812 | Sensitive | + | high | Insensitive |
| HCC1954 | Insensitive | + | high | Insensitive |
| HCC202 | Insensitive | + | low | Sensitive |
| MDA-MB-468 | Insensitive | - | high | Insensitive |
| HCC1569 | Insensitive | + | high | Insensitive |
| BT-20 | Insensitive | - | high | Insensitive |
| BT-483 | Insensitive | + | high | Insensitive |
| BT-549 | Insensitive | - | high | Insensitive |
| CAMA-1 | Insensitive | - | low | Sensitive |
| HCC1428 | Insensitive | - | high | Insensitive |
| HCC1806 | Insensitive | - | high | Insensitive |
| HCC1937 | Insensitive | - | high | Insensitive |
| HS578T | Insensitive | - | high | Insensitive |

(continued)

| Cell line | Reference example 1 | comparative example 1 | Example 1 | |
|---|---|---|---|---|
| | Sensitivity to lapatinib | Expression of HER2 | Inhibitory capacity of Src inhibitor | Determination results |
| MCF7 | Insensitive | - | high | Insensitive |
| MDA-MB-157 | Insensitive | - | high | Insensitive |
| MDA-MB-231 | Insensitive | - | high | Insensitive |
| MDA-MB-415 | Insensitive | - | high | Insensitive |
| MDA-MB-435S | Insensitive | - | high | Insensitive |
| MDA-MB-436 | Insensitive | - | high | Insensitive |
| T-47D | Insensitive | - | low | Sensitive |
| ZR-75-1 | Insensitive | - | high | Insensitive |

As is apparent from Table 9, the determination results by the determination method of the present embodiment almost correspond to the results of Reference example 1 (the conventional method) and Comparative example 1. This shows that the determination method of the present embodiment can determine sensitivity of tumor cells to the ATP competitive inhibitor for EGFR/HER2 without requiring a complicated process for proliferating cells and depending on any detection procedure which may affect the determination results due to characteristics like antibodies.

## Claims

**1.** A method for determining sensitivity of tumor cells to an ATP competitive inhibitor for EGFR and/or HER2 comprising steps of:

evaluating inhibitory capacity of an ATP competitive inhibitor for tyrosine kinase Src in a measurement sample obtained by preparing a cellular fraction containing tyrosine kinase from a biological sample containing tumor cells; and

determining that the tumor cells have sensitivity to an ATP competitive inhibitor for tyrosine kinase EGFR and/or HER2 when the inhibitory capacity of the ATP competitive inhibitor for Src is evaluated to be low and/or determining that the tumor cells do not have sensitivity to the ATP competitive inhibitor to EGFR and/or HER2 when the inhibitory capacity of the ATP competitive inhibitor for Src is evaluated to be high.

**2.** The method according to claim 1, wherein the tumor cells are breast cancer cells.

**3.** The method according to claim 1 or 2, wherein the cellular fraction is a membrane fraction.

**4.** The method according to any one of claims 1 to 3, wherein the ATP competitive inhibitor to EGFR and/or HER2 comprises N-(3-chloro-4-[[(3-fluorophenyl)methyl]oxy]phenyl)-6-[5-([[ 2-(methylsulfonyl)ethyl]amino]methyl)-2-furanyl]-4-quinazol ineamine bis(4-methylbenzenesulfonate)monohydrate;
N-[3-chlorc-4-[(3-fluorophenyl) methoxy]phenyl]-6-[5-[(2-methylsulfonylethylamino)methyl]-2 -furyl]quinazolin-4-amine;
N-[4-[(3-chloro-4-fluorophenyl)amino]-7-[[(3S)-tetrahydro-3 -furanyl]oxy]-6-quinazolinyl]-4-(dimethylamino)-2-butenamid e;
6-[4-[(4-ethyl-1-piperazinyl)methyl]phenyl]-N-[(1S)-1-pheny lethyl]-7H-pyrrolo[2,3-d]pyrimidin-4-amine;
(2E)-N-[4-[[3-chloro-4-(2-pyridinylmethoxy)phenyl]amino]-3-cyano-7-ethoxy-6-quinolinyl]-4-(dimethylamino)-2-butenamide ;
[4-[[1-(3-fluorophenyl)methyl]-1H-indazol-5-ylamino]-5-meth ylpyrrolo[2,1-f][1,2,4]triazine-6-yl]carbamic acid, (3S)-3-morpholinylmethylester;
N4-[3-chloro-4-(thiazol-2-ylmethoxy)phenyl]-N6-[(4R)-4-meth yl-4,5-dihydrooxazol-2-yl]quinazoline-4,6-diamine;
4-(4-benzyloxyanilino)-6,7-dimethoxyquinazoline;
N4-(1-benzyl-1H-indazol-5-yl)-N6,N6-dimethyl-pyrido-[3,4-d] -pyrimidine-4,6-diamine; or

N-[3-chloro-4-[(3-fluorophenyl)methoxy]phenyl]-6-[2-[[[2-(m ethylsulfonyl)ethyl]amino]methyl]-4-thiazolyl]-4-quinazolin eamine dihydrochloride.

5. The method according to any one of claims 1 to 4, wherein the ATP competitive inhibitor to Src comprises
4-(4'-phenoxyanilino)-6,7-dimethoxyquinazoline;
1-(1,1-dimethylethyl)-1-(4-methylphenyl)-1H-pyrazolo-[3,4-d ]pyrimidine-4-amine;
4-amino-1-tert-butyl-3-(1'-naphthyl)pyrazolo[3,4-d]pyrimidi ne;
4-amino-5-(4-chlorophenyl)-7-(t-butyl)pyrazolo[3,4-d]pyrimi dine;
2-oxo-3-(4,5,6,7-tetrahydro-1H-indole-2-yl methylene)-2,3-dihydro-1H-indole-5-sulfonic acid dimethyl amide; or
a compound represented by Structural formula below:

[Formula 1]

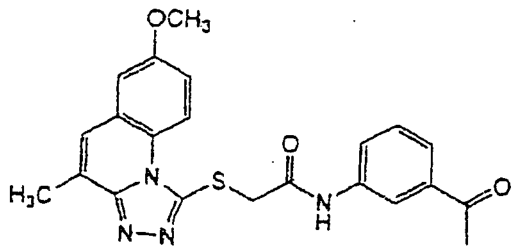

6. The method according to any one of claims 1 to 5, wherein the evaluation is performed by measuring the activity value of tyrosine kinase in the measurement sample in the presence of the ATP competitive inhibitor to Src and evaluating the inhibitory capacity of the ATP competitive inhibitor to Src based on the measured activity value.

7. The method according to claim 6, wherein performed the evaluation is performed by further measuring the activity value of tyrosine kinase in the measurement sample in the absence of the ATP competitive inhibitor to Src and evaluating the inhibitory capacity of the ATP competitive inhibitor to Src based on the activity values measured in the presence or absence of the ATP competitive inhibitor to Src.

8. The method according to claim 7, wherein
the evaluation is performed by evaluating the inhibitory capacity of the Src inhibitor based on a difference or a ratio of an activity value measured in the presence of the ATP competitive inhibitor to Src and an activity value measured in the absence of the ATP competitive inhibitor to Src.

9. The method according to claim 8, wherein
the evaluation is performed by evaluating the inhibitory capacity of the ATP competitive inhibitor to Src based on the results obtained by comparing a difference or a ratio of an activity value measured in the presence of the ATP competitive inhibitor to Src and an activity value measured in the absence of the ATP competitive inhibitor to Src with a threshold value.

10. A computer program product comprising: a computer readable medium, and software instructions, on the computer readable medium, for enabling a computer to perform operations comprising:

acquiring an activity value of tyrosine kinase in a measurement sample obtained from a biological sample containing tumor cells in the presence of an ATP competitive inhibitor for tyrosine kinase Src;
calculating a value of inhibitory capacity of the ATP competitive inhibitor for Src based on the activity value;
comparing the value of inhibitory capacity of the ATP competitive inhibitor for Src with a threshold value;
determining that the tumor cells have sensitivity to the ATP competitive inhibitor for tyrosine kinase EGFR and/or HER2 when the value of inhibitory capacity of the ATP competitive inhibitor for Src is lower than the threshold value based on the compared result and/or determining that the tumor cells do not have sensitivity to the ATP competitive inhibitor for EGFR and/or HER2 when the value of inhibitory capacity of the ATP competitive inhibitor for Src is more than the threshold value; and
outputting determination results.

11. The computer program product according to claim 10, wherein

the acquisition further comprises acquiring an activity value of tyrosine kinase in the measurement sample in the absence of the ATP competitive inhibitor to Src and the calculation comprises calculating a value showing the inhibitory capacity of the ATP competitive inhibitor to Src based on the activity values in the presence or absence of the ATP competitive inhibitor to Src.

**12.** The computer program product according to claim 11, wherein
the value of inhibitory capacity of the ATP competitive inhibitor to Src is a difference or a ratio of an activity value measured in the presence of the ATP competitive inhibitor for Src and an activity value measured in the absence of the ATP competitive inhibitor for Src.

**13.** The computer program product according to any one of claims 11 to 12, wherein the tumor cells are breast cancer cells.

**14.** The computer program product according to any one of claims 11 to 13, wherein
the ATP competitive inhibitor to EGFR and/or HER2 comprises
N-(3-chloro-4-[[(3-fluorophenyl)methyl]oxy]phenyl)-6-[5-([[ 2-(methylsulfonyl)ethyl]amino]methyl)-2-furanyl]-4-quinazol ineamine bis(4-methylbenzenesulfonate)monohydrate;
N-[3-chloro-4-[(3-fluorophenyl) methoxy]phenyl]-6-[5-[(2-methylsulfonylethylamino)methyl]-2 -furyl]quinazolin-4-amine;
N-[4-[(3-chloro-4-fluorophenyl)amino]-7-[[(3S)-tetrahydro-3 -furanyl]oxy]-6-quinazolinyl]-4-(dimethylamino)-2-butenamid e;
5-[4-[(4-ethyl-1-piperazinyl)methyi]phenyl]-N-[(1S)-1-pheny lethyl]-7H-pyrrolo[2,3-d]pyrimidin-4-amine;
(2E)-N-[4-[[3-chloro-4-(2-pyridinylmethoxy)phenyl]amino]-3-cyano-7-ethoxy-6-quinolinyl]-4-(dimethylamino)-2-butenamide ;
[4-[[1-(3-fluorophenyl)methyl]-1H-indazol-5-ylamino]-5-meth ylpyrrolo[2,1-f][1,2,4]triazine-6-yl]carbamic acid, (3S)-3-morpholinylmethylester;
N4-[3-chloro-4-(thiazol-2-ylmethoxy)phenyl]-N6-[(4R)-4-meth yl-4,5-dihydrooxazol-2-yl]quinazoline-4,6-diamine;
4-(4-benzyloxyanilino)-6,7-dimethoxyquinazoline;
N4-(1-benzyl-1H-indazol-5-yl)-N6,N6-dimethyl-pyrido-[3,4-d] -pyrimidine-4,6-diamine; or
N-[3-chloro-4-[(3-fluorophenyl)methoxy]phenyl]-6-[2-[[[2-(m ethylsulfonyl)ethyl]amino]methyl]-4-thiazolyl]-4-quinazolin eamine dihydrochloride.

**15.** The computer program product according to any one of claims 11 to 14, wherein
the ATP competitive inhibitor to Src comprises
4-(4'-phenoxyanilino)-6,7-dimethoxyquinazoline;
1-(1,1-dimethylethyl)-1-(4-methylphenyl)-1H-pyrazolo-[3,4-d ]pyrimidine-4-amine:
4-amino-1-tert-butyl-3-(1'-naphthyl)pyrazolo[3,4-d]pyrimidi ne;
4-amino-5-(4-chlorophenyl)-7-(t-butyl)pyrazolo[3,4-d]pyrimi dine; 2-oxo-3-(4,5,6,7-tetrahydro-1H-indole-2-yl methylene)-2,3-dihydro-1H-indole-5-sulfonic acid dimethylamide; or
a compound represented by Structural formula below:

[Formula 1]

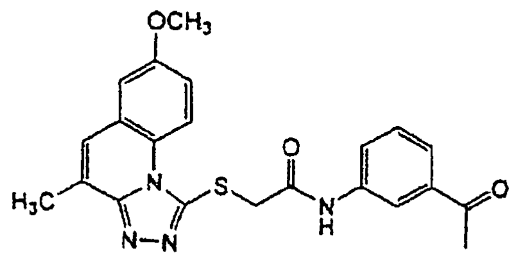

## Patentansprüche

**1.** Verfahren zum Bestimmen der Sensitivität von Tumorzellen gegenüber einem ATP-kompetitiven Inhibitor für EGFR und/oder HER2, das die Schritte umfasst:

Beurteilen der Inhibitionsfähigkeit eines ATP-kompetitiven Inhibitors für die Tyrosinkinase Src in einer Messprobe, die durch das Zubereiten einer Zellfraktion, die Tyrosinkinase enthält, aus einer biologischen Probe, die Tumorzellen enthält, hergestellt wurde; sowie
Bestimmen, dass die Tumorzellen Sensitivität gegenüber einem ATP-kompetitiven Inhibitor für die Tyrosinkinase EGFR und/oder HER2 aufweisen, wenn die Inhibitionsfähigkeit des ATP-kompetitiven Inhibitors für Src als niedrig bestimmt wird, und/oder Bestimmen, dass die Tumorzellen keine Sensitivität gegenüber dem ATP-kompetitiven Inhibitor für EGFR und/oder HER2 aufweisen, wenn die Inhibitionsfähigkeit des ATP-kompetitiven Inhibitors für Src als hoch beurteilt wird.

**2.** Verfahren gemäß Anspruch 1, wobei Tumorzellen Brustkrebszellen sind.

**3.** Verfahren gemäß Anspruch 1 oder 2, wobei die Zellfraktion eine Membranfraktion ist.

**4.** Verfahren gemäß einem der Ansprüche 1 bis 3, wobei der ATP-kompetitive Inhibitor für EGFR und/oder HER2
N-(3-chlor-4-[[(3-fluorphenyl)methyl]oxy]phenyl)-6-[5-([[2-(methylsulfonyl)ethyl]amino]methyl)-2-furanyl]4-chinazolinamin-bis(4-methylbenzolsulfonat)monohydrat;
N-(3-chlor-4-[[(3-fluorphenyl)methyloxy]phenyl)-6-[5-[(2-methylsulfonylethylamino)methyl]-2-furyl]-chinazolin-4-amin;
N-[4-[(chlor-4-fluorphenyl)amino]-7-[[(3S)-tetrahydro-3-furanyl]oxy]-6-chinazolinyl]-4-(dimethylamino)-2-butenamid;
6-[4-[(4-Ethyl-1-piperazinyl)methyl]phenyl]-N-[(1S)-1-Phenylethyl]-7H-pyrrol[2,3-d]pyrimidin-4-amin;
(2E)-N-[4-[[3-chlor-4-(2-pyridinylmethyoxy)phenyl]amino]-3-cyano-7-ethoxy-6-chinolinyl]-4-(dimethylamino)-2-butenamid;
[4-[[1-(3-Fluorphenyl)methyl]1H-indazol-5-ylaminol-5-methylpyrrolo[2,1-f][1,2,4]triazin-6-yl]carbaminsäure, (3S)-3-Morpholinylmethylester;
N4-[3-Chlor-4-(Thiazol-2-ylmethoxy)phenyl]-N6-[(4R)-4-methyl-4,5-dihydrooxazol-2-yl]chinazolin-4,6-diamin;
4-(4-Benzyloxyanilin)-6,7-dimethoxychinazolin;
N4-(1-Benzyl-1H-indazol-5-yl)-N6,N6-dimethylpyrido-[3,4-d]-pyrimidin-4,6-diamin; oder
N-[3-Chlor-4-[(3-fluorphenyl)methoxy]phenyl]-6-[2-[[[2-(methylsulfonyl)ethyl]amino]methyl]-4-thiazolyl]-4-chinazolinamindihydrochlorid umfasst.

**5.** Verfahren gemäß einem der Ansprüche 1 bis 4, wobei der ATP-kompetitive Inhibitor vfür Src
4-(4'-Phenoxyanilin)-6,7-dimethoxychinazolin;
1-(1,1-Dimethylethyl)-1-(4-methylphenyl)-1H-pyrazol-[3,4-d]pyrimidin-4-amin;
4-Amino-1-tert-butyl-3-[1'-naphthyl)pyrazol[3,4-d]pyrimidin;
4-Amino-5-(4-chlorphenyl)-7-(t-butyl)pyrazolo[3,4-d]pyrimidin;
2-Oxo-3-(4,5,6,7-tetrahydro-1H-indol-2-yl-methylen)-2,3-dihydro-1H-indol-5-sulfonsäuredimethylamid; oder
eine Verbindung, die durch die Strukturformel unten wiedergegeben wird:

[Formel 1]

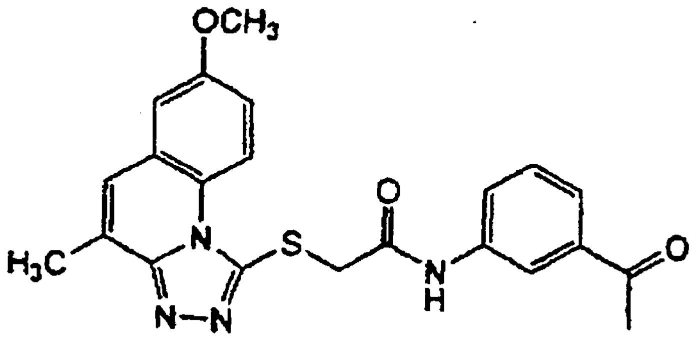

umfasst.

**6.** Verfahren gemäß einem der Ansprüche 1 bis 5, wobei die Beurteilung durch das weitere Messen des Aktivitätswerts von Tyrosinkinase in der Messprobe in Anwesenheit des ATP-kompetitiven Inhibitors für Src und das Beurteilen

der Inhibitionsfähigkeit des ATP-kompetitiven Inhibitors für Src basierend auf den Aktivitätswerten, die in der Gegenwart oder Abwesenheit des ATP-kompetitiven Inhibitors für Src gemessen wurden, durchgeführt wird.

**7.** Verfahren gemäß Anspruch 6, wobei die Beurteilung durch das weitere Messen des Aktivitätswerts von Tyrosinkinase in der Messprobe in Abwesenheit des ATP-kompetitiven Inhibitors für Src und das Beurteilen der Inhibitionsfähigkeit des ATP-kompetitiven Inhibitors für Src basierend auf den Aktivitätswerten, die in der Gegenwart oder Abwesenheit des ATP-kompetitiven Inhibitors für Src gemessen wurden, durchgeführt wird.

**8.** Verfahren gemäß Anspruch 7, wobei die Beurteilung durch das Beurteilen der Inhibitionsfähigkeit des Src-Inhibitors basierend auf einem Unterschied oder einem Verhältnis eines Aktivitätswerts, der in Gegenwart des ATP-kompetitiven Inhibitors für Src gemessen wurde, und eines Aktivitätswerts, der in Abwesenheit des ATP-kompetitiven Inhibitors für Src gemessen wurde, durchgeführt wird.

**9.** Verfahren gemäß Anspruch 8, wobei die Beurteilung durch das Beurteilen der Inhibitionsfähigkeit des ATP-kompetitiven Inhibitors für Src basierend auf den Ergebnissen, die durch das Vergleichen eines Unterschieds oder eines Verhältnisses eines Aktivitätswerts, der in Gegenwart des ATP-kompetitiven Inhibitors für Src gemessen wurde, und eines Aktivitätswerts, der in Abwesenheit des ATP-kompetitiven Inhibitors für Src mit einem Schwellenwert gemessen wurde, durchgeführt wird.

**10.** Computerprogrammprodukt, das umfasst: ein Computerlesbares Medium und Software-Anweisungen auf dem Computer-lesbaren Medium, die einem Computer ermöglichen, Arbeitsabläufe durchzuführen, die umfassen:

Erfassen eines Aktivitätswerts von Tyrosinkinase in einer Messprobe, die von einer biologischen Probe erhalten wurde, die Tumorzellen enthält, in Gegenwart eines ATP-kompetitiven Inhibitors für die Tyrosinkinase-Src;
Berechnen eines Werts der Inhibitionsfähigkeit des ATP-kompetitiven Inhibitors für Src basierend auf dem Aktivitätswert;
Vergleichen des Werts der Inhibitionsfähigkeit des ATP-kompetitiven Inhibitors für Src mit einem Schwellenwert;
Bestimmen, dass die Tumorzellen Sensitivität gegenüber dem ATP-kompetitiven Inhibitor für Tyrosinkinase EGFR und/oder HER2 aufweisen, wenn der Wert der Inhibitionsfähigkeit des ATP-kompetitiven Inhibitors für Src niedriger als der Schwellenwert ist, basierend auf dem verglichenen Ergebnis, und/oder Bestimmen, dass die Tumorzellen keine Sensitivität gegenüber dem ATP-kompetitiven Inhibitor für EGFR und/oder HER2 aufweisen, wenn der Wert der Inhibitionsfähigkeit des ATP-kompetitiven Inhibitors für Src mehr als der Schwellenwert ist; und
Ausgeben der Bestimmungsergebnisse.

**11.** Computerprogrammprodukt gemäß Anspruch 10, wobei
die Erfassung weiterhin das Erfassen eines Aktivitätswerts von Tyrosinkinase in der Messprobe in Abwesenheit des ATP-kompetitiven Inhibitors für Src umfasst, und die Berechnung das Berechnen eines Wertes umfasst, der die Inhibitionsfähigkeit des ATP-kompetitiven Inhibitors für Src basierend auf den Aktivitätswerten in Gegenwart oder Abwesenheit des ATP-kompetitiven Inhibitors für Src zeigt.

**12.** Computerprogrammprodukt gemäß Anspruch 11, wobei
der Wert der Inhibitionsfähigkeit des ATP-kompetitiven Inhibitors für Src ein Unterschied oder ein Verhältnis eines Aktivitätswerts, der in Gegenwart des ATP-kompetitiven Inhibitors für Src gemessen wurde, und eines Aktivitätswerts, der in Abwesenheit des ATP-kompetitiven Inhibitors für Src gemessen wurde, ist.

**13.** Computerprogrammprodukt gemäß einem der Ansprüche 11 bis 12, wobei die Tumorzellen Brustkrebszellen sind.

**14.** Computerprogrammprodukt gemäß einem der Ansprüche 11 bis 13, wobei
der ATP-kompetitive Inhibitor für EGFR und/oder HER2
N-(3-chlor-4-[[(3-fluorphenyl)methyl]oxy]phenyl)-6-[5-([[2-(methylsulfonyl)ethyl]amino]methyl)-2-furanyl]4-chinazolinamin-bis(4-methylbenzolsulfonat)monohydrat;
N-(3-chlor-4-[[(3-fluorphenyl)methyloxy]phenyl)-6-[5-[(2-methylsulfonylethylamino)methyl]-2-furyl]-chinazolin-4-amin;
N-[4-[(chlor-4-fluorphenyl)amino]-7-[[(3S)-tetrahydro-3-furanyl]oxy]-6-chinazolinyl]-4-(dimethylamino)-2-butenamid;
6-[4-[(4-Ethyl-1-piperazinyl)methyl]phenyl]-N-[(1S)-1-Phenylethyl]-7H-pyrrol[2,3-d]pyrimidin-4-amin;
(2E)-N-[4-[[3-chlor-4-(2-pyridinylmethyoxy)phenyl]amino]-3-cyano-7-ethoxy-6-chinolinyl]-4-(dimethylamino)-2-butenamid;
[4-[[1-(3-Fluorphenyl)methyl]1H-indazol-5-ylamino]-5-methylpyrrolo[2,1-f][1,2,4]triazin-6-yl]carbaminsäure, (3S)-3-Morpholinylmethylester;
N4-[3-Chlor-4-(Thiazol-2-ylmethoxy)phenyl]-N6-[(4R)-4-methyl-4,5-dihydrooxazol-2-yl]chinazolin-4,6-diamin;
4-(4-Benzyloxyanilin)-6,7-dimethoxychinazolin;
N4-(1-Benzyl-1H-indazol-5-yl)-N6,N6-dimethylpyrido-[3,4-d]pyrimidin-4,6-diamin; oder
N-[3-Chlor-4-[(3-fluorphenyl)methoxy]phenyl]-6-[2-[[[2-(methylsulfonyl)ethyl]amino]methyl]-4-thiazolyl]-4-chinazolinamindihydrochlorid umfasst.

**15.** Computerprogrammprodukt gemäß einem der Ansprüche 11 bis 14, wobei der ATP-kompetitive Inhibitor für Src
4-(4'-Phenoxyanilin)-6,7-dimethoxychinazolin;
1-(1,1-Dimethylethyl)-1-(4-methylphenyl)-1H-pyrazol-[3,4-d]pyrimidin-4-amin;
4-Amino-1-tert-butyl-3-[1'-naphthyl)pyrazol[3,4-d]pyrimidin;
4-Amino-5-(4-chlorphenyl)-7-(t-butyl)pyrazolo[3,4-d]pyrimidin;
2-Oxo-3-(4,5,6,7-tetrahydro-1H-indol-2-yl-methylen)-2,3-dihydro-1H-indol-5-sulfonsäuredimethylamid; oder
eine Verbindung, die durch die Strukturformel unten wiedergegeben wird:

[Formel 1]

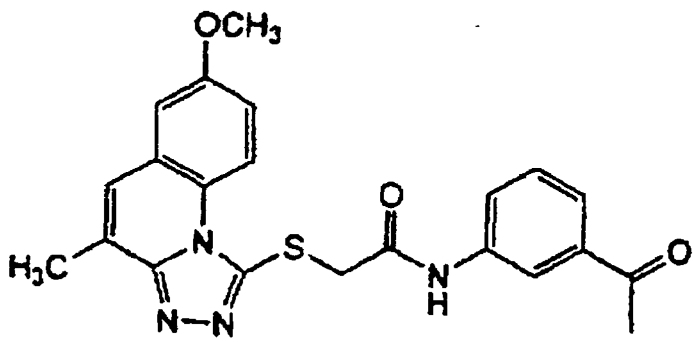

umfasst.

## Revendications

**1.** Procédé de détermination de la sensibilité de cellules tumorales à un inhibiteur de l'EGFR et/ou du HER2 concurrent de l'ATP comprenant les étapes suivantes :

l'évaluation de la capacité inhibitrice d'un inhibiteur du Src de tyrosine kinase concurrent de l'ATP dans un échantillon de mesure obtenu en préparant une fraction cellulaire contenant de la tyrosine kinase provenant d'un échantillon biologique contenant des cellules tumorales ; et
la détermination du fait que les cellules tumorales présentent une sensibilité à un inhibiteur de l'EGFR et/ou du HER2 de tyrosine kinase concurrent de l'ATP lorsqu'on évalue que la capacité inhibitrice de l'inhibiteur du Src concurrent de l'ATP est faible et/ou la détermination du fait que les cellules tumorales ne présentent pas de sensibilité à l'inhibiteur d'EGFR et/ou d'HER2 concurrent de l'ATP lorsqu'on évalue que la capacité inhibitrice de l'inhibiteur du Src concurrent de l'ATP est élevée.

**2.** Procédé selon la revendication 1, dans lequel les cellules tumorales sont des cellules de cancer du sein.

**3.** Procédé selon la revendication 1 ou 2, dans lequel la fraction cellulaire est une fraction membranaire.

**4.** Procédé selon l'une quelconque des revendications 1 à 3, dans lequel l'inhibiteur de l'EGFR et/ou du HER2 concurrent de l'ATP comprend
le bis(4-méthylbenzènesulfonate) de N-(3-chloro-4-[[(3-fluorophényl)méthyl]oxy]phényl)-6-[5-([[2-(méthylsulfonyl)éthyl]amino]méthyl)-2-furanyl]-4-quinazolineamine monohydraté ;
la N-[3-chloro-4-[(3-fluorophényl)méthoxy]phényl]-6-[5-[(2-méthylsulfonyléthylamino)méthyl]-2-furyl]-quinazolin-4-amine ;
le N-[4-[(3-chloro-4-fluorophényl)amino]-7-[[(3S)-tétrahydro-3-furanyl]oxy]-6-quinazolinyl]-4-(diméthylamino)-2-buténamide ;
la 6-[4-[(4-éthyl-1-pipérazinyl)méthyl]phényl]-N-[(1S)-1-phényléthyl]-7H-pyrrolo[2,3-d]pyrimidin-4-amine ;
le (2E)-N-[4-[[3-chloro-4-(2-pyridinylméthoxy)phényl]amino]-3-cyano-7-éthoxy-6-quinolinyl]-4-(diméthylamino)-2-buténamide ;
le (3S)-3-morpholinylméthylester de l'acide [4-[[1-(3-fluorophényl)méthyl]-1H-indazol-5-ylamino]-5-méthylpyrrolo[2,1-f][1,2,4]triazine-6-yl]carbamique ;
la N4-[3-chloro-4-(thiazol-2-ylméthoxy)phényl]-N6-[(4R)-4-méthyl-4,5-dihydrooxazol-2-yl]quinazoline-4,6-diamine ;
la 4-(4-benzyloxyanilino)-6,7-diméthoxyquinazoline ;
la N4-(1-benzyl-1H-indazol-5-yl)-N6,N6-diméthyl-pyrido-[3,4-d]pyrimidine-4,6-diamine ; ou
le dichlorhydrate de N-[3-chloro-4-[(3-fluorophényl)méthoxy]phényl]-6-[2-[[(2-(méthylsulfonyl)éthyl]amino]méthyl]-4-thiazolyl]-4-quinazolineamine.

**5.** Procédé selon l'une quelconque des revendications 1 à 4, dans lequel l'inhibiteur du Src concurrent de l'ATP comprend
la 4-(4'-phénoxyanilino)-6,7-diméthoxyquinazoline ;
la 1-(1,1-diméthyléthyl)-1-(4-méthylphényl)-1H-pyrazolo-[3,4-d]pyrimidine-4-amine ;
la 4-amino-1-tert-butyl-3-(1'-naphtyl)pyrazolo[3,4-d]pyrimidine ; la 4-amino-5-(4-chlorophényl)-7-(t-butyl)pyrazolo[3,4-d]pyrimidine ;
le diméthylamide de l'acide 2-oxo-3-(4,5,6,7-tétrahydro-1H-indole-2-ylméthylène)-2,3-dihydro-1H-indole-5-sulfonique ; ou
un composé représenté par la formule structurelle ci-dessous :

(Formule 1)

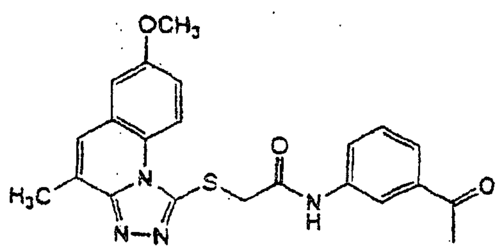

**6.** Procédé selon l'une quelconque des revendications 1 à 5, dans lequel l'évaluation est réalisée en mesurant la valeur d'activité de la tyrosine kinase dans l'échantillon de mesure en présence de l'inhibiteur du Src concurrent de l'ATP et en évaluant la capacité inhibitrice de l'inhibiteur du Src concurrent de l'ATP d'après la valeur d'activité mesurée.

**7.** Procédé selon la revendication 6, dans lequel l'évaluation est réalisée en mesurant en outre la valeur d'activité de la tyrosine kinase dans l'échantillon de mesure en l'absence de l'inhibiteur du Src concurrent de l'ATP et en évaluant la capacité inhibitrice de l'inhibiteur du Src concurrent de l'ATP d'après les valeurs d'activité mesurées en présence ou en l'absence de l'inhibiteur du Src concurrent de l'ATP.

**8.** Procédé selon la revendication 7, dans lequel l'évaluation est réalisée en évaluant la capacité inhibitrice de l'inhibiteur du Src d'après la différence ou le rapport de la valeur d'activité mesurée en présence de l'inhibiteur du Src concurrent de l'ATP et de la valeur d'activité mesurée en l'absence de l'inhibiteur du Src concurrent de l'ATP.

**9.** Procédé selon la revendication 8, dans lequel l'évaluation est réalisée en évaluant la capacité inhibitrice de l'inhibiteur du Src concurrent de l'ATP d'après les résultats obtenus en comparant la différence ou le rapport de la valeur d'activité mesurée en présence de l'inhibiteur du Src concurrent de l'ATP et de la valeur d'activité mesurée en l'absence de l'inhibiteur du Src concurrent de l'ATP avec une valeur seuil.

**10.** Produit de programme informatique comprenant : un support lisible par ordinateur et des instructions de logiciel, sur le support lisible par ordinateur, pour permettre à un ordinateur de réaliser les opérations comprenant :

l'acquisition d'une valeur d'activité de la tyrosine kinase dans un échantillon de mesure obtenu à partir d'un échantillon biologique contenant des cellules tumorales en présence d'un l'inhibiteur du Src de la tyrosine kinase concurrent de l'ATP ;
le calcul d'une valeur de capacité inhibitrice de l'inhibiteur du Src concurrent de l'ATP d'après la valeur d'activité ;
la comparaison de la valeur de capacité inhibitrice de l'inhibiteur du Src concurrent de l'ATP avec une valeur seuil ;
la détermination du fait que les cellules tumorales présentent une sensibilité à l'inhibiteur de l'EGFR et/ou du HER2 de tyrosine kinase concurrent de l'ATP lorsque la valeur de la capacité inhibitrice de l'inhibiteur du Src concurrent de l'ATP est inférieure à la valeur seuil d'après le résultat comparé et/ou la détermination du fait que les cellules tumorales ne présentent pas de sensibilité à l'inhibiteur d'EGFR et/ou d'HER2 concurrent de l'ATP lorsque la valeur de la capacité inhibitrice de l'inhibiteur du Src concurrent de l'ATP est supérieure à la valeur seuil ; et
la sortie des résultats de la détermination.

**11.** Produit de programme informatique selon la revendication 10, dans lequel l'acquisition comprend en outre l'acquisition de la valeur d'activité de la tyrosine kinase dans l'échantillon de mesure en l'absence de l'inhibiteur du Src concurrent de l'ATP et le calcul comprend le calcul d'une valeur représentant la capacité inhibitrice de l'inhibiteur du Src concurrent de l'ATP d'après les valeurs d'activité en présence ou en l'absence de l'inhibiteur du Src concurrent de l'ATP.

**12.** Produit de programme informatique selon la revendication 11, dans lequel la valeur de la capacité inhibitrice de l'inhibiteur du Src concurrent de l'ATP est une différence ou un rapport de la valeur d'activité mesurée en présence de l'inhibiteur du Src concurrent de l'ATP et de la valeur d'activité mesurée en l'absence de l'inhibiteur du Src concurrent de l'ATP.

**13.** Produit de programme informatique selon l'une quelconque des revendications 11 et 12, dans lequel les cellules tumorales sont des cellules de cancer du sein.

**14.** Produit de programme informatique selon l'une quelconque des revendications 11 à 13, dans lequel l'inhibiteur de l'EGFR et/ou du HER2 concurrent de l'ATP comprend
le bis(4-méthylbenzènesulfonate) de N-(3-chloro-4-[[(3-fluorophényl)méthyl]oxy]phényl)-6-[5-([[2-(méthylsulfonyl) éthyl]amino]méthyl)-2-furanyl]-4-quinazolineamine monohydraté ;
la N-[3-chloro-4-[(3-fluorophényl)méthoxy]phényl]-6-[5-[(2-méthylsulfonyléthylamino)méthyl]-2-furyl]-quinazolin-4-amine ;
le N-[4-[(3-chloro-4-fluorophényl)amino]-7-[[(3S)-tétrahydro-3-furanyl]oxy]-6-quinazolinyl]-4-(diméthylamino)-2-buténamide ;
la 6-[4-[(4-éthyl-1-pipérazinyl)méthyl]phényl]-N-[(1S)-1-phényléthyl]-7H-pyrrolo[2,3-d]pyrimidin-4-amine ;
le (2E)-N-[4-[[3-chloro-4-(2-pyridinylméthoxy)phnéyl]amino]-3-cyano-7-éthoxy-6-quinolinyl]-4-(diméthylamino)-2-buténamide ;
le (3S)-3-morpholinylméthylester de l'acide [4-[[1-(3-fluorophényl)méthyl]-1H-indazol-5-ylamino]-5-méthylpyrrolo [2,1-f][1,2,4]triazine-6-yl]carbamique ;
la N4-[3-chloro-4-(thiazol-2-ylméthoxy)phényl]-N6-[(4R)-4-méthyl-4,5-dihydrooxazol-2-yl]quinazoline-4,6-diamine ;
la 4-(4-benzyloxyanilino)-6,7-diméthoxyquinazoline ;
la N4-(1-benzyl-1H-indazol-5-yl)-N6,N6-diméthyl-pyrido-[3,4-d]pyrimidine-4,6-diamine ; ou
le dichlorhydrate de N-[3-chloro-4-[(3-fluorophényl)méthoxylphényl]-6-[2-[[(2-(méthylsulfonyl)éthyl]amino]méthyl]-4-thiazolyl] -4-quinazolineamine.

**15.** Produit de programme informatique selon l'une quelconque des revendications 11 à 14, dans lequel
l'inhibiteur du Src concurrent de l'ATP comprend
la 4-(4'-phénoxyanilino)-6,7-diméthoxyquinazoline ;
la 1-(1,1-diméthyléthyl)-1-(4-méthylphényl)-1H-pyrazolo-[3,4-d]pyrimidine-4-amine ;
la 4-amino-1-tert-butyl-3-(1'-naphtyl)pyrazolo[3,4-d]pyrimidine ; la 4-amino-5-(4-chlorophényl)-7-(t-butyl)pyrazolo [3,4-d]pyrimidine ;
le diméthylamide de l'acide 2-oxo-3-(4,5,6,7-tétrahydro-1H-indole-2-ylméthylène)-2,3-dihydro-1H-indole-5-sulfonique ; ou
un composé représenté par la formule structurelle ci-dessous :

(Formule 1)

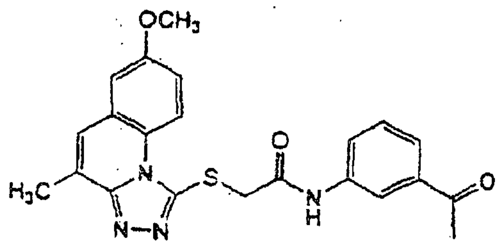

FIG. 1

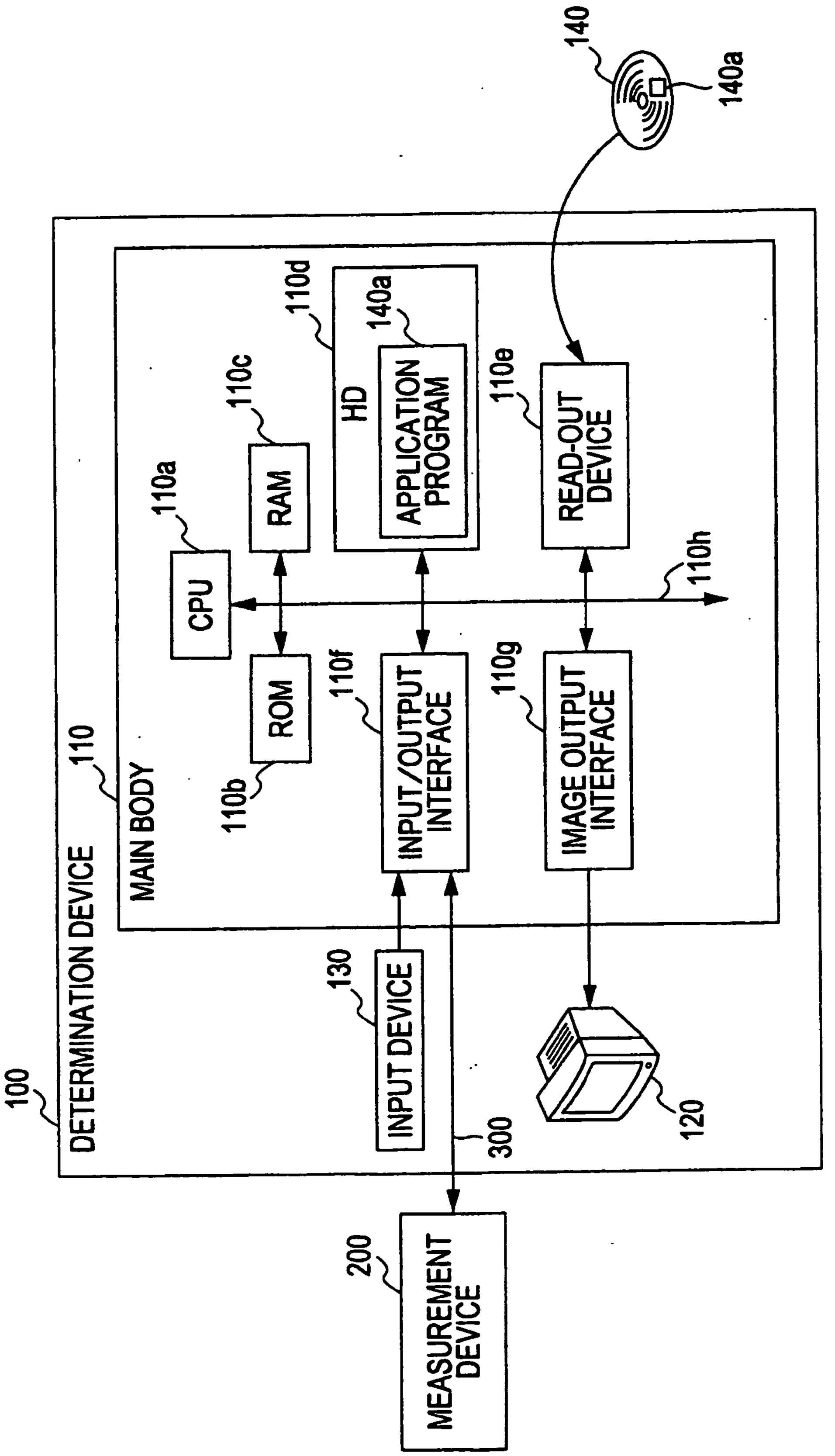
100
DETERMINATION DEVICE
110
MAIN BODY
110a
CPU
110b
ROM
110c
RAM
110d
HD
140a
APPLICATION PROGRAM
110e
READ-OUT DEVICE
110f
INPUT/OUTPUT INTERFACE
110g
IMAGE OUTPUT INTERFACE
110h
130
INPUT DEVICE
120
140
140a
300
200
MEASUREMENT DEVICE

FIG. 2

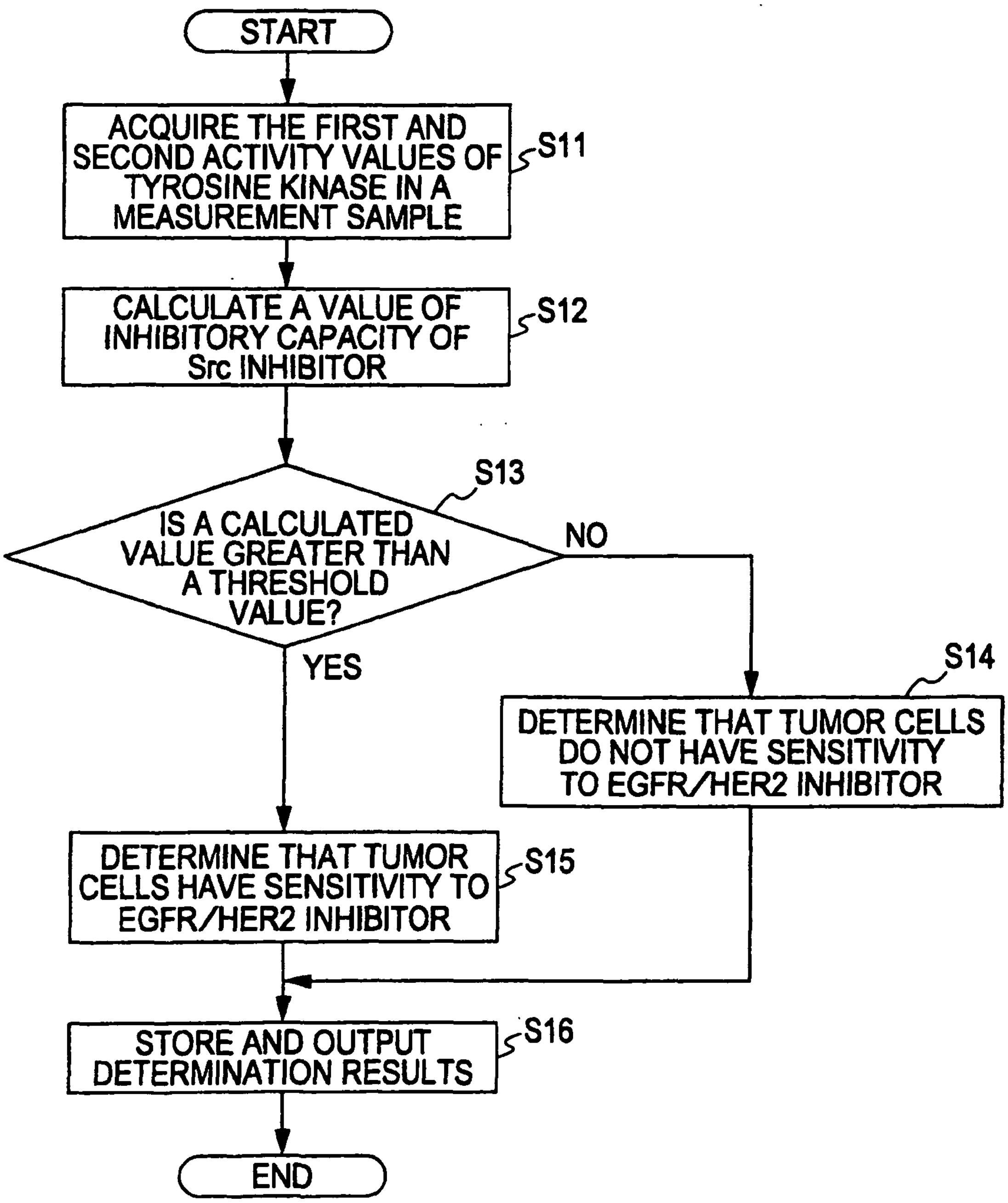
START
ACQUIRE THE FIRST AND SECOND ACTIVITY VALUES OF TYROSINE KINASE IN A MEASUREMENT SAMPLE
S11
CALCULATE A VALUE OF INHIBITORY CAPACITY OF Src INHIBITOR
S12
IS A CALCULATED VALUE GREATER THAN A THRESHOLD VALUE?
S13
NO
YES
DETERMINE THAT TUMOR CELLS DO NOT HAVE SENSITIVITY TO EGFR/HER2 INHIBITOR
S14
DETERMINE THAT TUMOR CELLS HAVE SENSITIVITY TO EGFR/HER2 INHIBITOR
S15
STORE AND OUTPUT DETERMINATION RESULTS
S16
END

FIG. 3

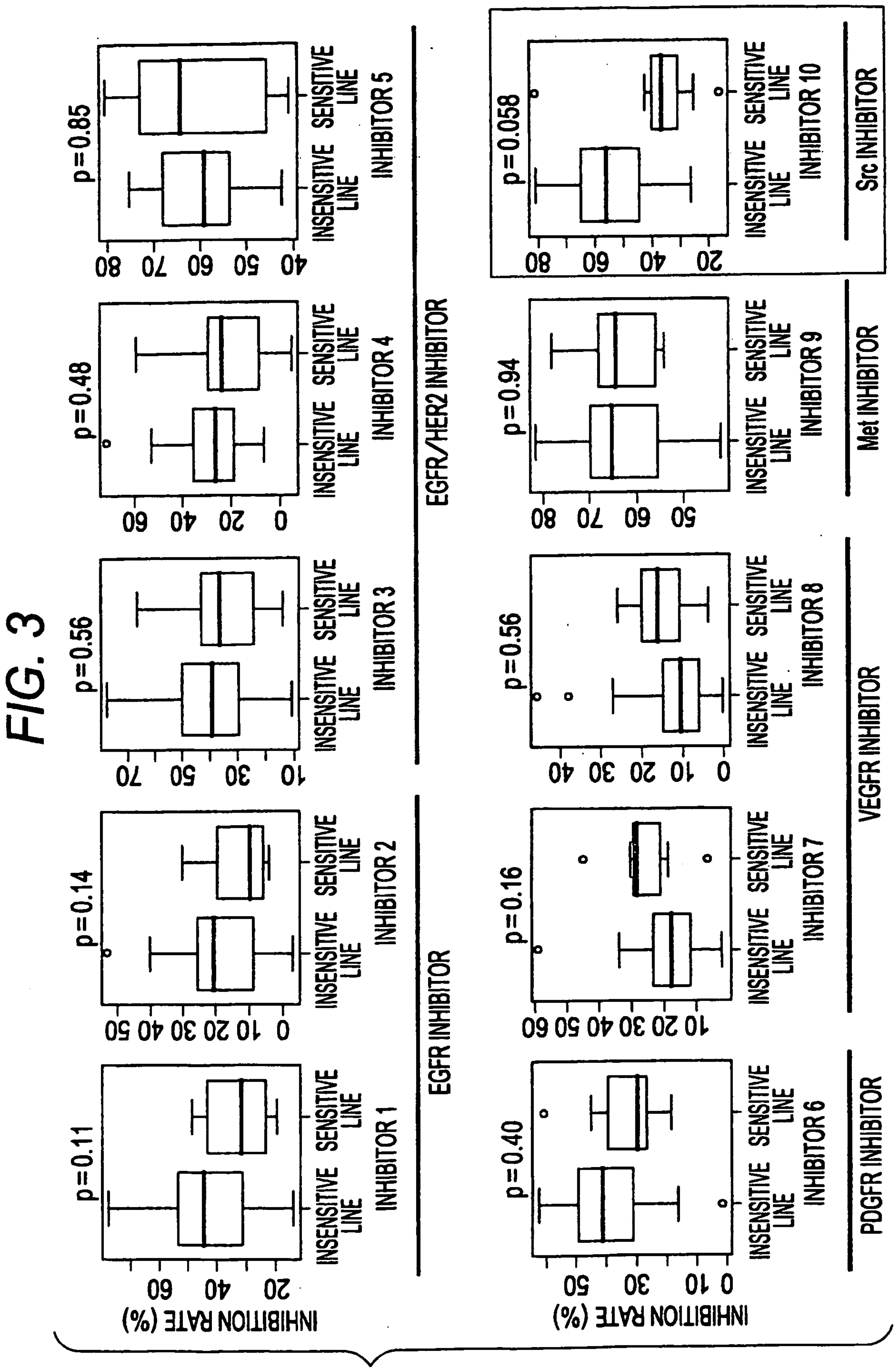
INHIBITION RATE (%)
p=0.11
INSENSITIVE LINE
SENSITIVE LINE
INHIBITOR 1
p=0.14
INHIBITOR 2
EGFR INHIBITOR
p=0.56
INHIBITOR 3
p=0.48
INHIBITOR 4
p=0.85
INHIBITOR 5
EGFR/HER2 INHIBITOR
p=0.40
INHIBITOR 6
PDGFR INHIBITOR
p=0.16
INHIBITOR 7
p=0.56
INHIBITOR 8
VEGFR INHIBITOR
p=0.94
INHIBITOR 9
Met INHIBITOR
p=0.058
INHIBITOR 10
Src INHIBITOR

*FIG. 4*

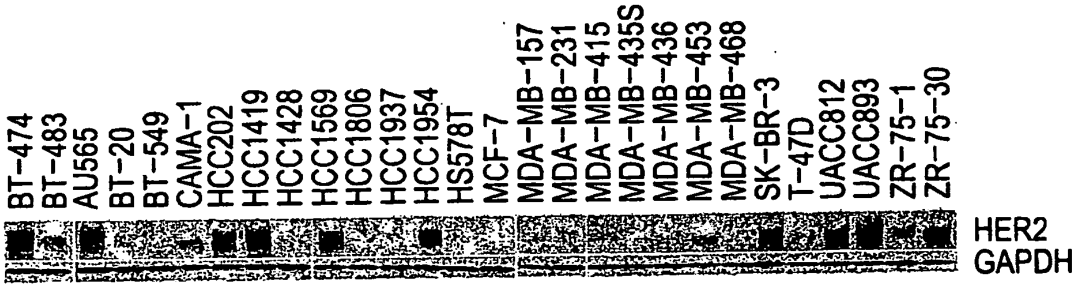
BT-474
BT-483
AU565
BT-20
BT-549
CAMA-1
HCC202
HCC1419
HCC1428
HCC1569
HCC1806
HCC1937
HCC1954
HS578T
MCF-7
MDA-MB-157
MDA-MB-231
MDA-MB-415
MDA-MB-435S
MDA-MB-436
MDA-MB-453
MDA-MB-468
SK-BR-3
T-47D
UACC812
UACC893
ZR-75-1
ZR-75-30
HER2
GAPDH

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011027809 A
- WO 2008127718 A
- WO 2007106432 A
- WO 2008089135 A


### Non-patent literature cited in the description


- **GOTTFRIED E. KONECNY et al.** *Cancer Res,* 2006, vol. 66, 1630-1639
- **LANXI SONG et al.** *Cancer Res,* 2006, vol. 66, 5542-5548
- **NORIO SASAKI et al.** *The Journal of Biological Chemistry,* 1985, vol. 260, 9793-9804
- **SERGEI BRAUN et al.** *The Journal of Biological Chemistry,* 1984, vol. 259, 2051-2054
- **M. ABDEL-GHANY et al.** *Proceeding of The National Academy of Science,* 1990, vol. 87, 7061-7065
- **TONY HUNTER.** *The Journal of Biological Chemistry,* 1992, vol. 257, 4843-4848
- **BLAZAR et al.** *Journal of Immunology,* 1997, vol. 159, 5821-5833
- **BIRD et al.** *Science,* 1988, vol. 242, 423-426